# EUROPEAN PATENT APPLICATION

(11) **EP 2 221 358 A1**
(43) Date of publication of application: **25.08.2010**
(21) Application number: 09153499.0
(22) Date of filing: 24.02.2009
(51) Int. Cl.: C12N 1/16, C12N 15/81

(54) **Biotin-prototrophic yeasts**

(71) Applicant: Universität für Bodenkultur Wien, 1180 Wien (AT)
(72) Inventor: Mattanovich, Diethard, 1120, Vienna (AT); Gasser, Brigitte, 1050, Vienna (AT)
(74) Representative: Redl, Gerda

(57) **Abstract**

The invention refers to a host cell line of a biotin prototrophic yeast comprising a BIO1 gene and a xenogene, a method of producing such a host cell line comprising the steps of:
a) providing a yeast host cell,
b) providing a BIO1 gene and a xenogene,
c) introducing said genes into said host cell, and
d) cultivating said host cell in a biotin-deficient medium to obtain a host cell line,

and a method of producing a recombinant xenogenic polypeptide.

SEQUENCE LISTING

<110> Universität für Bodenkultur Wien

<120> Biotin-prototrophic yeasts

<130> BK1/EP

<160> 15

<170> PatentIn version 3.3

<210> 1
<211> 3561
<212> DNA
<213> artificial

<220>
<223> pPM2d_ZeoloxP_pTPI

<400> 1
ccggtgaatt cgtgatctga agcttcgtac gctgcaggtc gacaaccctt aatataactt 60

cgtataatgt atgctatacg aagttattag gtctagatcg gtaccacaca ccatagcttc 120

aaaatgtttc tactcctttt ttactcttcc agattttctc ggactccgcg catcgccgta 180

ccacttcaaa acacccaagc acagcatact aaatttcccc tctttcttcc tctagggtgt 240

cgttaattac ccgtactaaa ggtttggaaa agaaaaaaga gaccgcctcg tttctttttc 300

ttcgtcgaaa aaggcaataa aaatttttat cacgtttctt tttcttgaaa tttttttttt 360

tagttttttt ctctttcagt gacctccatt gatatttaag ttaataaacg gtcttcaatt 420

tctcaagttt cagtttcatt tttcttgttc tattacaact ttttttactt cttgttcatt 480

agaaagaaag catagcaatc taatctaagg gcggtgttga caattaatca tcggcatagt 540

atatcggcat agtataatac gacaaggtga ggaactaaac catggccaag ttgaccagtg 600

ccgttccggt gctcaccgcg cgcgacgtcg ccggagcggt cgagttctgg accgaccggc 660

tcgggttctc ccgggacttc gtggaggacg acttcgccgg tgtggtccgg gacgacgtga 720

ccctgttcat cagcgcggtc caggaccagg tggtgccgga caacaccctg gcctgggtgt 780

gggtgcgcgg cctggacgag ctgtacgccg agtggtcgga ggtcgtgtcc acgaacttcc 840

gggacgcctc cgggccggcc atgaccgaga tcggcgagca gccgtggggg cgggagttcg 900

ccctgcgcga cccggccggc aactgcgtgc acttcgtggc cgaggagcag gactgacacg 960

tccgacggcg gcccacgggt cccaggcctc ggagatccgt cccccttttc ctttgtcgat 1020

atcatgtaat tagttatgtc acgcttacat tcacgccctc cccccacatc cgctctaacc 1080

gaaaaggaag gagttagaca acctgaagtc taggtcccta tttatttttt tatagttatg 1140

ttagtattaa gaacgttatt tatatttcaa atttttcttt tttttctgta cagacgcgtg 1200

tacgcatgta acattatact gaaaaccttg cttgagaagg ttttgggacg ctcgaaggct 1260

ttaatttgca agctggagac caacatgtga gcaaggtacc attcgagaac ccttaatata 1320

acttcgtata atgtatgcta tacgaagtta ttaggtgata tcagatccac tagatctgaa 1380

ttcgagctct gagcaaaagg ccagcaaaag gccaggaacc gtaaaaaggc cgcgttgctg 1440

gcgtttttcc ataggctccg cccccctgac gagcatcaca aaaatcgacg ctcaagtcag 1500
aggtggcgaa acccgacagg actataaaga taccaggcgt ttccccctgg aagctccctc 1560
gtgcgctctc ctgttccgac cctgccgctt accggatacc tgtccgcctt tctcccttcg 1620
ggaagcgtgg cgctttctca atgctcacgc tgtaggtatc tcagttcggt gtaggtcgtt 1680
cgctccaagc tgggctgtgt gcacgaaccc cccgttcagc ccgaccgctg cgccttatcc 1740
ggtaactatc gtcttgagtc caacccggta agacacgact tatcgccact ggcagcagcc 1800
actggtaaca ggattagcag agcgaggtat gtaggcggtg ctacagagtt cttgaagtgg 1860
tggcctaact acggctacac tagaagaaca gtatttggta tctgcgctct gctgaagcca 1920
gttaccttcg gaaaaagagt tggtagctct tgatccggca aacaaaccac cgctggtagc 1980
ggtggttttt ttgtttgcaa gcagcagatt acgcgcagaa aaaaaggatc tcaagaagat 2040
cctttgatct tttctacggg agctcttaat taagcggccg cttaattaag ggcccaagcc 2100
acgccggcgc caaggccctt caacgagaca ctcttccgtc agttccaaaa ccataagttt 2160
gccgatgtgt tggtccttgt aacgcatgga atttgggcca gggtattttt gatgaaatgg 2220
ttcagatggt ctgtggagga gtttgaaggc ttacgaaata taccacattg ccagtttata 2280
cagatggtta agggtgaaaa tcaacgttac accttgacga ccccattatt acgatggcgt 2340
gaaggagatg aagaccgggt agaagaaata agaaaagcgg tacagtttag gtccggagat 2400
ctagggaagg aggccttagc ttatattgta gctgctgaga gagaggcagc tgctggaaga 2460
tctgaaggcc ctatcacgta tgatgatggt gatgaccatt agagaacgcc cagagattga 2520
tagccagttc ttggacaaca attcggaact ttattcacgg tgcaaacatg atttgtgtgg 2580
atagcttcaa gtcagacatt tcatctcatc ccccttttta ctgctgctaa tcaccgttag 2640
tccgacagtt actctaatca atatttatta gtgttttagt tgcgcaaaac tcgagcctct 2700
tttccttatc tcttgacact tcctggagtc gaagtttttc agcgcaaatt cactctacaa 2760
tgtctaccga tactagaccg cctatcttcc ccctctaaat agcctattgg aagggtgcaa 2820
taaggtatat aaatctggcg cgattccccc ggacttttat gatccacatc acctcatctt 2880
actgccctca ctctctttcc tgatcctccc aggtccaccg atttcctcac tatcgtcgga 2940
tttctccttc cagcgcccta gagaattccg taaccaccgc aaaaatagca gcccccccct 3000
cacccatttt tttatttaaa agaacacctt actggcccgt tttcgtttct cctttactac 3060
aattgatttt taattttcag ttttttttca ttgatataca agatctatca caaacacaca 3120
cctgcaggcc atgactagtt ctgcacctta agtctagaga acaaaaactc atctcagaag 3180
aggatctgaa tagcgccgtc gaccatcatc atcatcatca ttgaggccgc ctcggccgga 3240
tccacgtccg acggcggccc acgggtccca ggcctcggag atccgtcccc cttttccttt 3300
gtcgatatca tgtaattagt tatgtcacgc ttacattcac gccctccccc cacatccgct 3360
ctaaccgaaa aggaaggagt tagacaacct gaagtctagg tccctattta tttttttata 3420
gttatgttag tattaagaac gttatttata tttcaaattt ttcttttttt tctgtacaga 3480

cgcgtgtacg catgtaacat tatactgaaa accttgcttg agaaggtttt gggacgctcg 3540

aaggctttaa tttgcaagct a 3561

<210> 2
<211> 6705
<212> DNA
<213> artificial

<220>
<223> pPM2d_pPGI_BIO6_pPGK_BIO1 expression vector

<400> 2
ccggtgaatt cgtgatctga agcttcgtac cgagctctga gcaaaaggcc agcaaaaggc 60

caggaaccgt aaaaaggccg cgttgctggc gtttttccat aggctccgcc cccctgacga 120

gcatcacaaa aatcgacgct caagtcagag gtggcgaaac ccgacaggac tataaagata 180

ccaggcgttt ccccctggaa gctccctcgt gcgctctcct gttccgaccc tgccgcttac 240

cggatacctg tccgcctttc tcccttcggg aagcgtggcg ctttctcaat gctcacgctg 300

taggtatctc agttcggtgt aggtcgttcg ctccaagctg ggctgtgtgc acgaaccccc 360

cgttcagccc gaccgctgcg ccttatccgg taactatcgt cttgagtcca acccggtaag 420

acacgactta tcgccactgg cagcagccac tggtaacagg attagcagag cgaggtatgt 480

aggcggtgct acagagttct tgaagtggtg gcctaactac ggctacacta gaagaacagt 540

atttggtatc tgcgctctgc tgaagccagt taccttcgga aaaagagttg gtagctcttg 600

atccggcaaa caaaccaccg ctggtagcgg tggttttttt gtttgcaagc agcagattac 660

gcgcagaaaa aaaggatctc aagaagatcc tttgatcttt tctacgggag ctcttaatta 720

agcggccgct taattaaggg cccaagccac gccggcgcca aggcccagca ctttctctgg 780

agattagtaa tcgatggtca ggatgggaac cgaatgtctt tggctggcct ttccccaaat 840

ttgagaacaa aattcaattg ctgaggtaat acttttttcc ccatacaaga aaggccatcg 900

tgtaattatc tcttaccttc tactattcga gaagttgtaa ccaaatggct gataacatcg 960

aaaccatccg tcctggaatc aactaccagg acaaactatt gaccgaaata gacattcttg 1020

atgatgttaa acaactgtca acgcagatgg aacaggaagg gaaaggattc gtcttcccta 1080

aagatcatta ctccaagata aaatcactaa aggggttgca agtgaagctg ttgaaagaaa 1140

tgcaagagtt ggctgaaatt cagcagtcta ggcatcaaga cgaaactgca tacaggcaga 1200

agcttcaaga cttggaagat actacatcag aacttagaaa cgttgctttg acgtaatagg 1260

tcacctgtgt tactagatgt cggatgtctg tgattgcagt aacacccggc attcggtcag 1320

ctgtctacct ttcaggatat cctcccccca ccgagctcgg acgcctttct cgcccaccca 1380

ccacgcaaag ttccgcccac cttatacttt gtggaagtct tttcgccgcg caccacactt 1440

gcccgttcgc agaaaaagaa attccaggta gcccttattc cgtcaggcaa taaatatata 1500

agcgattgca gacaatctga cgtcccatcc ccttgtctat ttaaacctcc taggttgctt 1560

aaatttaaaa tctattcgag tcccaacctt tccattcttc cggaataatt caactccaac 1620

caattgataa acacctgcag gatgcttgaa catcaattaa cccaagaaga tctcgaattt 1680
gataaaaaac acatatggca cccgtacaca tcaataacta ctccattgaa agtgtaccct 1740
attaccaaag cagaaggtag ttatttgtac ctcgataatg gtaccaaggt agtagatggt 1800
atggcaagct ggtggtgcgt tcagcaagga tacaacaatc atcgcctaaa tgcagccgcc 1860
atttcacaga ttaataaaat gtcacatgtg atgtttggtg gaattactca taaggctgga 1920
attgattttt gtaaaaagtt gctcgctttg ttaccagata ccctggaatg tgctttactg 1980
gcggattctg ggtctatttc tgtcgacatt gcaatgaaga tggctttaag atatcatcat 2040
tcattgggga atacaacgaa aaaaagattc ttgacaatta agaagggcta ccacggtgac 2100
gcttttggag ccgtatcagt ttgtgaccct gtcaattcaa ggcataatac ttataacggg 2160
tttctcgctg aaaacatatt ctgcaaggct ccagaagtcc gctttgattg tcgtgaaaag 2220
gacgttgaga aactggtaga ggaactcgac gtaaagccat ttgctgagat tattgataaa 2280
caccacagtg aaatttctgg tgttgtgatg gaaagcatcg tacagggcgc tggtggtcta 2340
agaatgtatc atccatattt cctgaaacgt gtccgtgcac tctgcaacga ctttaatatt 2400
ttgttgattc ttgatgaagt tgcagtgggg cttggaagaa cgggtatgct gtttggattt 2460
gagcatgcag ggatagtgcc tgacatagtc tgtctaggta agactttgac ggccggttat 2520
ttgacgttat ctgcaacagt taccaccaga gagattggtg atcagatttc cagtggacct 2580
gagggttgct tcatgcacgg acaaacctat atggcaaatc cgttagcatg cgctgttgca 2640
agtgaaaact tgtcgatctt aatggaaggt aaatggaaat ctcaagttcg gcaaatagaa 2700
gtccagctga agaaagagct ggtgccctta ctggaacacc ccatcgtggc tgatgtaaga 2760
attctagggg ctattggtgt tgtcgaggtt acaaaacgtg tgaacgtcga agtcttacaa 2820
gagaaattta ttaaggcagg tgcgtggatt agacctttcg gtaacataat ctacattcta 2880
cctccataca taattacttc cgaggagttg accgtcttaa cagaagctat cagatctgtt 2940
ctagacttta tttagggccg cctcggccgg atccacgtcc gacggcggcc cacgggtccc 3000
aggcctcgga gatccgtccc cctttccttt gtcgatatc atgtaattag ttatgtcacg 3060
cttacattca cgccctcccc ccacatccgc tctaaccgaa aaggaaggag ttagacaacc 3120
tgaagtctag gtccctattt atttttttat agttatgtta gtattaagaa cgttatttat 3180
atttcaaatt tttctttttt ttctgtacag acgcgtgtac gcatgtaaca ttatactgaa 3240
aaccttgctt gagaaggttt tgggacgctc gaaggcttta atttgcaagc taccggcgcc 3300
aaggcccaag ttggtaccca gccgatcacg cctgctctga gtttggctgg agcagcaaat 3360
ctcatgagat tatcaaaaag gatcttcacc tagatccttt taaattaaaa atgaagtttt 3420
aaatcaatct aaagtatata tgagtaaact tggtctgaca gttaccaatg cttaatcagt 3480
gaggcaccta tctcagcgat ctgtctattt cgttcatcca tagttgcctg actccccgtc 3540
gtgtagataa ctacgatacg ggagggctta ccatctggcc ccagtgctgc aatgataccg 3600
cgagacccac gctcaccggc tccagattta tcagcaataa accagccagc cggaagggcc 3660

gagcgcagaa gtggtcctgc aactttatcc gcctccatcc agtctattaa ttgttgccgg 3720
gaagctagag taagtagttc gccagttaat agtttgcgca acgttgttgc cattgctgca 3780
ggcatcgtgg tgtcacgctc gtcgtttggt atggcttcat tcagctccgg ttcccaacga 3840
tcaaggcgag ttacatgatc ccccatgttg tgcaaaaaag cggttagctc cttcggtcct 3900
ccgatcgttg tcagaagtaa gttggccgca gtgttatcac tcatggttat ggcagcactg 3960
cataattctc ttactgtcat gccatccgta agatgctttt ctgtgactgg tgagtactca 4020
accaagtcat tctgagaata gtgtatgcgg cgaccgagtt gctcttgccc ggcgtcaaca 4080
cgggataata ccgcgccaca tagcagaact ttaaaagtgc tcatcattgg aaaacgttct 4140
tcggggcgaa aactctcaag gatcttaccg ctgttgagat ccagttcgat gtaacccact 4200
cgtgcaccca actgatcttc agcatctttt actttcacca gcgtttctgg gtgagcaaaa 4260
acaggaaggc aaaatgccgc aaaaaaggga ataagggcga cacggaaatg ttgaatactc 4320
atactcttcc tttttcaata ttattgaagc atttatcagg gttattgtct catgataacc 4380
gaggtttaaa ttaaggtaca taacaaaaat tcaatgttca aagacgcaca taccaagact 4440
tactaatcgc agaatgttgg tgcagtattt gtcgtaagcc aaaaccatcg atgttgactt 4500
cctaattcag tctttaaacc gcaaaaggat tctgattcgc agatggcctg atctccaaac 4560
tcaggctggg gctctaactc gagcaagtgt cctatgctgt aggccgcagc ccttttggtt 4620
cgacgacgtg cgtggttatg agacgctcgg ctgttttgcg ctaagctggc cgtatcgagt 4680
aaattctaca ggcacctgcg aggcaagcaa tctactaatg tttatttttc gtccaaccta 4740
attgtggttt caaagcgcta tcaggtgggg ggtaagagga atgtgagtgg aaagcgaaaa 4800
taactggcag ctggggtcag atcccgtgat gccacctctt gtggtatttt gaaacgcgtg 4860
ttgcgattgg ccgcgagaac ggaaaggaat atatttactg ccgatcgcat tttggcctca 4920
aataaatctt gagcttttgg acatagatta tatgttcttt cttggaagct ctttcagcta 4980
atagtgaagt gtttcctact aaggatcgcc tccaaacgtt ccaactacgg gcggaggttg 5040
caaagaaaac gggtctctca gcgaattgtt ctcatccatg agtgagtcct ctccgtcctt 5100
tcctcgcgcc tggcaataaa gcctccttcg gaggagctcc gtctagagaa taattgctgc 5160
ctttctgact ttcggactag cgccaaccgc gaaccacacc accacaccat cactgtcacc 5220
cgtcatagtt catccctctc tccttataaa gcatctaata ggttccacaa ttgtttgcca 5280
aaaaatctct tagcatagcc caattgatta cgaaacacct gcaggccatg aacacaaaat 5340
cactcgactt ttatgaacca cttgaaattg atggtcaaaa atacattaag atgactaaga 5400
aggaagacct aggcgtatat gaggccggag tgacacagga agcatttact gccaaggata 5460
agtatgatta caaaggaatt gtagagaatt tggaaaggta tgggctttgt atagttccaa 5520
gctttataga accatccaaa tgtgatcaga tcttagagga gctcgatccc catttctaca 5580
gacacgacgc atggcagggt tcaccattcc caaaggaaac aacggtggtg acaagagcag 5640
tcctccactc tcctactgtc ttgaaggagg ttgtgtctga ccgtctgttt tgcgatatgg 5700

caaaccattt tttaaatgag gaaaattact tcttgactgg aaatgtcatc aggaagtgtt 5760

caagtggtat tcagctaaac tctggtattg tttacaaggt tggtgctggt gctggtgatc 5820

agggctacca tcgagaagac catatccatc ataccattca tcaagcacgt gacagtttcc 5880

aatacggaaa ggaaacgcta ctcggagtag gggttgcttt cacggatatg aacaaagcta 5940

atggggctac gcgtgtgatt atcggttcgc acttgtgggg cccacatgat tcgtgtggga 6000

aattcgacaa gaggatggag tttcatgtaa atgctgccaa gggtgacgcg gttttgtttt 6060

taggcagcct ttaccatgcc gcaagtgcga accataccct agaagataga attgcgggat 6120

atttctttat gtcacagggc tacttgaaac aagaggaaaa tcttcacttt ggaacagacc 6180

ctgaattctt caaagacatg ccactagaga ccttgaaact cttgggattg acgaccagtg 6240

agccatattg tggtcacata gattacaaaa gtccaggaca tattgcgaac cccaacttgt 6300

ttgaaaatga agttgagaaa ggatactacg gtgagacaat caagattatt tatgacgata 6360

aagagtaagg ccgcctcggc cggatccacg tccgacggcg gcccacgggt cccaggcctc 6420

ggagatccgt cccccctttc ctttgtcgat atcatgtaat tagttatgtc acgcttacat 6480

tcacgccctc cccccacatc cgctctaacc gaaaaggaag gagttagaca acctgaagtc 6540

taggtcccta tttatttttt tatagttatg ttagtattaa gaacgttatt tatatttcaa 6600

atttttcttt tttttctgta cagacgcgtg tacgcatgta acattatact gaaaaccttg 6660

cttgagaagg ttttgggacg ctcgaaggct ttaatttgca agcta 6705

<210> 3
<211> 6509
<212> DNA
<213> artificial

<220>
<223> pPM2d_pPGK_BIO3_pPGI_BIO4 expression vector

<400> 3
ccggtgaatt cgtgatctga agcttcgtac cgagctctga gcaaaaggcc agcaaaaggc 60

caggaaccgt aaaaaggccg cgttgctggc gtttttccat aggctccgcc cccctgacga 120

gcatcacaaa aatcgacgct caagtcagag gtggcgaaac ccgacaggac tataaagata 180

ccaggcgttt ccccctggaa gctccctcgt gcgctctcct gttccgaccc tgccgcttac 240

cggatacctg tccgcctttc tcccttcggg aagcgtggcg ctttctcaat gctcacgctg 300

taggtatctc agttcggtgt aggtcgttcg ctccaagctg ggctgtgtgc acgaaccccc 360

cgttcagccc gaccgctgcg ccttatccgg taactatcgt cttgagtcca acccggtaag 420

acacgactta tcgccactgg cagcagccac tggtaacagg attagcagag cgaggtatgt 480

aggcggtgct acagagttct tgaagtggtg gcctaactac ggctacacta gaagaacagt 540

atttggtatc tgcgctctgc tgaagccagt taccttcgga aaaagagttg gtagctcttg 600

atccggcaaa caaaccaccg ctggtagcgg tggttttttt gtttgcaagc agcagattac 660

gcgcagaaaa aaaggatctc aagaagatcc tttgatcttt tctacgggag ctcttaatta 720

agcggccgct taattaaggg cccaagccac gccggcgcca aggcccaagt tggtacccag 780
ccgatcacgc ctgctctgag tttggctgga gcagcaaatc tcatgagatt atcaaaaagg 840
atcttcacct agatcctttt aaattaaaaa tgaagtttta aatcaatcta aagtatatat 900
gagtaaactt ggtctgacag ttaccaatgc ttaatcagtg aggcacctat ctcagcgatc 960
tgtctatttc gttcatccat agttgcctga ctccccgtcg tgtagataac tacgatacgg 1020
gagggcttac catctggccc cagtgctgca atgataccgc gagacccacg ctcaccggct 1080
ccagatttat cagcaataaa ccagccagcc ggaagggccg agcgcagaag tggtcctgca 1140
actttatccg cctccatcca gtctattaat tgttgccggg aagctagagt aagtagttcg 1200
ccagttaata gtttgcgcaa cgttgttgcc attgctgcag gcatcgtggt gtcacgctcg 1260
tcgtttggta tggcttcatt cagctccggt tcccaacgat caaggcgagt tacatgatcc 1320
cccatgttgt gcaaaaaagc ggttagctcc ttcggtcctc cgatcgttgt cagaagtaag 1380
ttggccgcag tgttatcact catggttatg gcagcactgc ataattctct tactgtcatg 1440
ccatccgtaa gatgcttttc tgtgactggt gagtactcaa ccaagtcatt ctgagaatag 1500
tgtatgcggc gaccgagttg ctcttgcccg gcgtcaacac gggataatac cgcgccacat 1560
agcagaactt taaaagtgct catcattgga aaacgttctt cggggcgaaa actctcaagg 1620
atcttaccgc tgttgagatc cagttcgatg taacccactc gtgcacccaa ctgatcttca 1680
gcatctttta ctttcaccag cgtttctggg tgagcaaaaa caggaaggca aaatgccgca 1740
aaaaagggaa taagggcgac acggaaatgt tgaatactca tactcttcct ttttcaatat 1800
tattgaagca tttatcaggg ttattgtctc atgataaccg aggtttaaat taaggtacat 1860
aacaaaaatt caatgttcaa agacgcacat accaagactt actaatcgca gaatgttggt 1920
gcagtatttg tcgtaagcca aaaccatcga tgttgacttc ctaattcagt ctttaaaccg 1980
caaaaggatt ctgattcgca gatggcctga tctccaaact caggctgggg ctctaactcg 2040
agcaagtgtc ctatgctgta ggccgcagcc cttttggttc gacgacgtgc gtggttatga 2100
gacgctcggc tgttttgcgc taagctggcc gtatcgagta aattctacag gcacctgcga 2160
ggcaagcaat ctactaatgt ttatttttcg tccaacctaa ttgtggtttc aaagcgctat 2220
caggtggggg gtaagaggaa tgtgagtgga aagcgaaaat aactggcagc tggggtcaga 2280
tcccgtgatg ccacctcttg tggtattttg aaacgcgtgt tgcgattggc cgcgagaacg 2340
gaaaggaata tatttactgc cgatcgcatt ttggcctcaa ataaatcttg agcttttgga 2400
catagattat atgttctttc ttggaagctc tttcagctaa tagtgaagtg tttcctacta 2460
aggatcgcct ccaaacgttc caactacggg cggaggttgc aaagaaaacg ggtctctcag 2520
cgaattgttc tcatccatga gtgagtcctc tccgtccttt cctcgcgcct ggcaataaag 2580
cctccttcgg aggagctccg tctagagaat aattgctgcc tttctgactt tcggactagc 2640
gccaaccgcg aaccacacca ccacaccatc actgtcaccc gtcatagttc atccctctct 2700
ccttataaag catctaatag gttccacaat tgtttgccaa aaaatctctt agcatagccc 2760

aattgattac gaaacacctg caggccatgt cccaagaaat ttcatataca ccagatgtcg 2820
cggaactgct agactttgat aaaaaacata tttggcatcc ttatacgtca ttgagttcac 2880
cattgaacgt ttatccggta aagagtgctc acggatgcaa gctggtgttg gataccgact 2940
caccagtgga cgtcgaggtg atcgacgcca tgtcgtcgtg gtggtgtgtt attcacgggt 3000
acaataatcc agaattaaat gaggccctta ccaagcagat gttaaagttt tctcacgtcc 3060
ttcttggtgg attcacccat aaaggagctg tgaatctagt ccaaaaactt ttaaaagtga 3120
ttgacgaacc ctctttgcag tactgtttcc tcgcagattc tggctcagta gcagtggaag 3180
tagctttgaa gatggcttta caatcaaaca tgtcagggga agctactaaa aatagaacaa 3240
aatttttgac aatcaagaac gggtatcatg gtgatacatt tggcgctatg agtgtttgtg 3300
atcctgagaa ttctatgcat catatctaca acgatcgact cagtgagaac atcttcgccc 3360
aagctccctc tattgtcgat ggtcttccaa ccagccagaa cggtttcgag gaccattgga 3420
atgcagaaga agttaccgat ctcaagaagc aatttgaatt gcatagtgat gagatctgtg 3480
ctgtgatttt ggagcctatc ttacagggtg caggaggttt gcgaccatac catccacagt 3540
tcctaatcga agttcagaaa ctatgtaacc aatacgacgt tttattcatc atggacgaga 3600
ttgctacggg atttggtaga acaggtgaga ttttcgcatt caagcactgt caaaaatacc 3660
aggaccaacg tggcatcagt ccttccgatc agatcaaagt tgttcctgac atattgtgcg 3720
ttgggaaggg cttaaccagt ggatatatga ctatgagtgc agtggtagtc aacgacaaag 3780
ttgcttccag aatctcttct ccaaatagcc ctactggtgg ctgcttcatg catggcccca 3840
ccttcatggg caatgccctg gcatgcagtg tggcagaaaa gtctatggat atactactgc 3900
gtggcgagtg gagaaaacaa gtgtctgcaa ttgaagacca aatttataga gaactatacc 3960
agtacatcaa gaacccagat aatgggctca ttggaactgt tgttaagcgt gtgtcagtta 4020
ttggagctgt aggtatcgta gagctataca agaagacaga tcccgagtgg ttccagaaaa 4080
agtttatatc caagggagta catatccgac ctttcaactg cctgtgttac attatgccac 4140
catatgtgat cacgacagag gaattgacga aggttaatca agtcttgatt gaagtattac 4200
atgagtggaa atcccacata aaccaatgag gccgcctcgg ccggatccac gtccgacggc 4260
ggcccacggg tcccaggcct cggagatccg tccccctttt cctttgtcga tatcatgtaa 4320
ttagttatgt cacgcttaca ttcacgccct ccccccacat ccgctctaac cgaaaaggaa 4380
ggagttagac aacctgaagt ctaggtccct atttattttt ttatagttat gttagtatta 4440
agaacgttat ttatatttca aatttttctt ttttttctgt acagacgcgt gtacgcatgt 4500
aacattatac tgaaaacctt gcttgagaag gttttgggac gctcgaaggc tttaatttgc 4560
aagctaccgg cgccaaggcc cagcactttc tctggagatt agtaatcgat ggtcaggatg 4620
ggaaccgaat gtctttggct ggcctttccc caaatttgag aacaaaattc aattgctgag 4680
gtaatacttt tttccccata caagaaaggc catcgtgtaa ttatctctta ccttctacta 4740
ttcgagaagt tgtaaccaaa tggctgataa catcgaaacc atccgtcctg gaatcaacta 4800

ccaggacaaa ctattgaccg aaatagacat tcttgatgat gttaaacaac tgtcaacgca 4860
gatggaacag gaagggaaag gattcgtctt ccctaaagat cattactcca agataaaatc 4920
actaaagggg ttgcaagtga agctgttgaa agaaatgcaa gagttggctg aaattcagca 4980
gtctaggcat caagacgaaa ctgcatacag gcagaagctt caagacttgg aagatactac 5040
atcagaactt agaaacgttg ctttgacgta ataggtcacc tgtgttacta gatgtcggat 5100
gtctgtgatt gcagtaacac ccggcattcg gtcagctgtc tacctttcag gatatcctcc 5160
ccccaccgag ctcggacgcc tttctcgccc acccaccacg caaagttccg cccaccttat 5220
actttgtgga agtcttttcg ccgcgcacca cacttgcccg ttcgcagaaa aagaaattcc 5280
aggtagccct tattccgtca ggcaataaat atataagcga ttgcagacaa tctgacgtcc 5340
catccccttg tctatttaaa cctcctaggt tgcttaaatt taaaatctat tcgagtccca 5400
acctttccat tcttccggaa taattcaact ccaaccaatt gataaacacc tgcaggccat 5460
gaacagcaaa tctcaacaac aagaacaaca accaattgta ttcgtcaccg gtacagacac 5520
tgatgttggt aaaacctttg tatcaacatt attggtacac aaatggaaag ctgcatactg 5580
gaaacccgta caaactggaa ttgagtcaga tcaaggtgac tccgagacat tgaaaaattt 5640
caaaatagcc gcatcaactt ggcaaccacc tatattcacg cccacttatg cgctgcagaa 5700
gcctctatct ccgctccagg ccatggagta cgaacctaat gtcgacatca ggttgttgga 5760
tttcgtagtt cctgaagagt ggagcgcaga gaatccattg gtcgtagaag gagccggcgg 5820
ggtttgcgtt cccatcactc gtaaattgga aattacgacg gatctaatta aacatctgat 5880
tgagactagc ggccatccag tgtacgtagt tgtcgtggca cgcagcgggc tagggactct 5940
gaatcataca ttgctgactt ggaatcatct ttgcgataat ggcttgagaa gccacctttt 6000
tggggtcatt ctcaatgggg aaccgaatga aggcaacgtg caggccctga agaagttcgg 6060
tgtcaatatt atggcacagg ttgcacaatg tactacggca catgatcagg atatggcgtt 6120
gcatgaactg ccatcggtgg agtcattaat gacccaacaa gacgtagaat agggccgcct 6180
cggccggatc cacgtccgac ggcggcccac gggtcccagg cctcggagat ccgtccccct 6240
tttcctttgt cgatatcatg taattagtta tgtcacgctt acattcacgc cctcccccca 6300
catccgctct aaccgaaaag gaaggagtta gacaacctga agtctaggtc cctatttatt 6360
tttttatagt tatgttagta ttaagaacgt tatttatatt tcaaattttt cttttttttc 6420
tgtacagacg cgtgtacgca tgtaacatta tactgaaaac cttgcttgag aaggttttgg 6480
gacgctcgaa ggctttaatt tgcaagcta 6509

<210> 4
<211> 1041
<212> DNA
<213> artificial

<220>
<223> BIO1 gene

<400> 4
atgaacacaa aatcactcga cttttatgaa ccacttgaaa ttgatggtca aaaatacatt 60

aagatgacta agaaggaaga cctaggcgta tatgaggccg gagtgacaca ggaagcattt 120

actgccaagg ataagtatga ttacaaagga attgtagaga atttggaaag gtatgggctt 180

tgtatagttc caagctttat agaaccatcc aaatgtgatc agatcttaga ggagctcgat 240

ccccatttct acagacacga cgcatggcag ggttcaccat tcccaaagga aacaacggtg 300

gtgacaagag cagtcctcca ctctcctact gtcttgaagg aggttgtgtc tgaccgtctg 360

ttttgcgata tggcaaacca ttttttaaat gaggaaaatt acttcttgac tggaaatgtc 420

atcaggaagt gttcaagtgg tattcagcta aactctggta ttgtttacaa ggttggtgct 480

ggtgctggtg atcagggcta ccatcgagaa gaccatatcc atcataccat tcatcaagca 540

cgtgacagtt tccaatacgg aaaggaaacg ctactcggag taggggttgc tttcacggat 600

atgaacaaag ctaatggggc tacgcgtgtg attatcggtt cgcacttgtg gggcccacat 660

gattcgtgtg ggaaattcga caagaggatg gagtttcatg taaatgctgc caagggtgac 720

gcggttttgt ttttaggcag cctttaccat gccgcaagtg cgaaccatac cctagaagat 780

agaattgcgg gatatttctt tatgtcacag ggctacttga aacaagagga aaatcttcac 840

tttggaacag accctgaatt cttcaaagac atgccactag agaccttgaa actcttggga 900

ttgacgacca gtgagccata ttgtggtcac atagattaca aaagtccagg acatattgcg 960

aaccccaact tgtttgaaaa tgaagttgag aaaggatact acggtgagac aatcaagatt 1020

atttatgacg ataaagagta a 1041

<210> 5
<211> 1191
<212> DNA
<213> artificial

<220>
<223> BIO2 gene

<400> 5
atgaacagac agtcgttgat ggcaggcagg agactcttct ccaaagtcag agtggtcaga 60

aaaccatcca cgttggccaa cgagttgggg tctttgtacg agcataacca accaattttt 120

gagcctccat cccctgaaaa aaaggtgtcc gctttagact atgcattttc tttgaaatct 180

ccggtttcaa cttggactaa agaacagctt agagagattt acaacactcc tctgatggaa 240

cttgttcacc gtgctcagat atatcatcgc aattttcaca atccttcgga agttcaaatt 300

tgcaacttgg tgaatatcaa gtcgggaggt tgtactgagg actgcaagta ctgttcccaa 360

tcggtgaaca atgacaccaa cttgaaagct gaaaagctta tgaccgtgga tgaggttgtt 420

gagcaggcta agattgccaa atccaacggc gcaacaagag tctgtttagg agcagcatac 480

agaactgtta gcaccagaaa gagagcgtta gaaagaatca ccacaatggt gaaggaagtc 540

aacaagctcg gcctggaaag ttgtgtcaca ctcggaatga ttgaagaaga acatgctaga 600

aagcttaagg acgcaggatt gaccgcatac aaccacaaca tcgacacctc aagagaacac 660

tatcctaaca tcattactac gagaacttat gatgatagac tgaagacgat tgacaacgtc 720

cagaaggccg gaatcagtgc ttgtaccggt ggaattctag gtcttggaga gacagtggaa 780

gaccacattg gatttctgta caccctgtgt accatgaaac agcaccctga atcactgccg 840

atcaacagat tggttccaat taaaggaact cccatcgcag acgagttggc ccgtgacaac 900

tccaagaagc ttaaattcga gtcaatgttg agaaccattg ccactgccag aatggtgatg 960

ccaaccagta ttatcagatt ggcagctggt agatacacta tgaaagagac agagcaattc 1020

ttatgcttta tggctggatg caatgcaatc tttagcggaa agaagatgtt gactactatg 1080

tgcaatgggt ttgatgagga taaagaactt ttagccaaat ggggtatgaa accgatgcag 1140

agtttcgcta gtaaacgtcc ttcgtcaatc gtggaaactt gccatatatg a 1191

<210> 6
<211> 1443
<212> DNA
<213> artificial

<220>
<223> BIO3 gene

<400> 6
atgtcccaag aaatttcata tacaccagat gtcgcggaac tgctagactt tgataaaaaa 60

catatttggc atccttatac gtcattgagt tcaccattga acgtttatcc ggtaaagagt 120

gctcacggat gcaagctggt gttggatacc gactcaccag tggacgtcga ggtgatcgac 180

gccatgtcgt cgtggtggtg tgttattcac gggtacaata atccagaatt aaatgaggcc 240

cttaccaagc agatgttaaa gttttctcac gtccttcttg gtggattcac ccataaagga 300

gctgtgaatc tagtccaaaa acttttaaaa gtgattgacg aaccctcttt gcagtactgt 360

ttcctcgcag attctggctc agtagcagtg gaagtagctt tgaagatggc tttacaatca 420

aacatgtcag gggaagctac taaaaataga acaaaatttt tgacaatcaa gaacgggtat 480

catggtgata catttggcgc tatgagtgtt tgtgatcctg agaattctat gcatcatatc 540

tacaacgatc gactcagtga gaacatcttc gcccaagctc cctctattgt cgatggtctt 600

ccaaccagcc agaacggttt cgaggaccat tggaatgcag aagaagttac cgatctcaag 660

aagcaatttg aattgcatag tgatgagatc tgtgctgtga ttttggagcc tatcttacag 720

ggtgcaggag gtttgcgacc ataccatcca cagttcctaa tcgaagttca gaaactatgt 780

aaccaatacg acgttttatt catcatggac gagattgcta cgggatttgg tagaacaggt 840

gagattttcg cattcaagca ctgtcaaaaa taccaggacc aacgtggcat cagtccttcc 900

gatcagatca aagttgttcc tgacatattg tgcgttggga agggcttaac cagtggatat 960

atgactatga gtgcagtggt agtcaacgac aaagttgctt ccagaatctc ttctccaaat 1020

agccctactg gtggctgctt catgcatggc cccaccttca tgggcaatgc cctggcatgc 1080

agtgtggcag aaaagtctat ggatatacta ctgcgtggcg agtggagaaa acaagtgtct 1140

gcaattgaag accaaattta tagagaacta taccagtaca tcaagaaccc agataatggg 1200

ctcattggaa ctgttgttaa gcgtgtgtca gttattggag ctgtaggtat cgtagagcta 1260

tacaagaaga cagatcccga gtggttccag aaaaagttta tatccaaggg agtacatatc 1320

cgacctttca actgcctgtg ttacattatg ccaccatatg tgatcacgac agaggaattg 1380

acgaaggtta atcaagtctt gattgaagta ttacatgagt ggaaatccca cataaaccaa 1440

tga 1443

<210> 7
<211> 714
<212> DNA
<213> artificial

<220>
<223> BIO4 gene

<400> 7
atgaacagca aatctcaaca acaagaacaa caaccaattg tattcgtcac cggtacagac 60

actgatgttg gtaaaacctt tgtatcaaca ttattggtac acaaatggaa agctgcatac 120

tggaaacccg tacaaactgg aattgagtca gatcaaggtg actccgagac attgaaaaat 180

ttcaaaatag ccgcatcaac ttggcaacca cctatattca cgcccactta tgcgctgcag 240

aagcctctat ctccgctcca ggccatggag tacgaaccta atgtcgacat caggttgttg 300

gatttcgtag ttcctgaaga gtggagcgca gagaatccat tggtcgtaga aggagccggc 360

ggggtttgcg ttcccatcac tcgtaaattg gaaattacga cggatctaat taaacatctg 420

attgagacta gcggccatcc agtgtacgta gttgtcgtgg cacgcagcgg gctagggact 480

ctgaatcata cattgctgac ttggaatcat ctttgcgata atggcttgag aagccacctt 540

tttggggtca ttctcaatgg ggaaccgaat gaaggcaacg tgcaggccct gaagaagttc 600

ggtgtcaata ttatggcaca ggttgcacaa tgtactacgg cacatgatca ggatatggcg 660

ttgcatgaac tgccatcggt ggagtcatta atgacccaac aagacgtaga atag 714

<210> 8
<211> 1686
<212> DNA
<213> artificial

<220>
<223> BIO5 gene

<400> 8
atgaacagag taggggcagt gttcctattt gtatatgaaa gaaatttttt tttgtctatt 60

gttccagatc gtcacaggac ggaaataaga atgtctagtt cagaaaggtc agaagtcaag 120

tttgacaagc actttaattg gtggtcccta ttaggtatcg cgttctcatt aagttgctca 180

tgggtcggta tctcagcgtc gatggccgtt ggtattgcca gtggagggcc actgcttatc 240

atctatgggt tgataattgc tgcttttttc agtctcatgt gtggtatatc tctgggagat 300

tttgctgcta tcctgccaaa cagcagcggt ggttcatttt gggttcttaa aatgttggaa 360

caagaatcag tcactttgaa aacccctgag tacgaggacc cttctgacga tgatgaagaa 420

gtgttcctcg agaattattg tcaaactttc aacgtggaag tttcttctaa atttcaaaag 480

gtttcttcca tggttgtagg gttgctgaac tatttcggtg ccatttttac taccgcaagt 540

atctgctcat ctttgtcaat gagctgtatc ggtattcata agttgttgca tccggactac 600

gagttaaagc actggcatgt ttttgtgggt tacgagtgta tcaatgccgt tttgacactt 660

ttcaacattt attcaactcc gttgccctat atctctcagt ttgggctcta tacgtctcta 720

ttgtctttcg ccatgacttt cattatttgt attgtttcaa gatctgacaa caccgtagac 780

ccatggccca aagcttctaa tatatttggg agctttgata atcaaacagg ctggaattcg 840

tctggaatgg cgtttgttgt cggtttggtc aatccgatct gggcatttgt tggcattgac 900

tctgctacgc acatgattga tgaagtgggt tatagtaagt cacgtttctt agttcccaaa 960

gttattatca ctaccatcat tgtgggcttt gtaactagtt ttatttattg cgttggtttg 1020

tttttctgca tcactgacca aacggcagtc gttgagtcta ttttacctat cgtagaaata 1080

ttttaccagg ccactggtaa taggaatctc agtgtcttcc tgcaatgtat gtgtatcaca 1140

acaggttttg tttcaggcat tgcaagtgga acctggcaaa gtcgaatcct ccaatcattc 1200

gggaaaagtt acgcaccatt ttataaagag ggttccttgg gaaacaaatc cttgaagaag 1260

ctagcagtgt tgacacctgg gtttaagtct ccattatatg cccatttttt gtcacaaata 1320

tgtgtaacga taattggctg tatatttatg ggatctagta ccgctttcaa cgcaattatc 1380

actgcatgca tcaccctgtt actaatgtcg tacgcagttc cttcttttat attcctgttc 1440

gttattaaaa aggaaaaatt cattcataga atcgagagtg acgtaaattg tgtcagcagg 1500

cctaaccgtc gtcgtatgtc tatgattcct catattatat gtattctatg gaccttgttc 1560

tgcctggtat ttttatcttt cccatataca ctaccggtta ctgcaggaaa catgaattac 1620

acctcagtag tgtatgcagt agttttctgc attatcagca tcgttgtttt ccctacatgc 1680

atctaa 1686

<210> 9
<211> 1314
<212> DNA
<213> artificial

<220>
<223> BIO6 gene

<400> 9
atgcttgaac atcaattaac ccaagaagat ctcgaatttg ataaaaaaca catatggcac 60

ccgtacacat caataactac tccattgaaa gtgtacccta ttaccaaagc agaaggtagt 120

tatttgtacc tcgataatgg taccaaggta gtagatggta tggcaagctg gtggtgcgtt 180

cagcaaggat acaacaatca tcgcctaaat gcagccgcca tttcacagat taataaaatg 240

tcacatgtga tgtttggtgg aattactcat aaggctggaa ttgatttttg taaaaagttg 300

ctcgctttgt taccagatac cctggaatgt gctttactgg cggattctgg gtctatttct 360

gtcgacattg caatgaagat ggctttaaga tatcatcatt cattggggaa tacaacgaaa 420

aaaagattct tgacaattaa gaagggctac cacggtgacg cttttggagc cgtatcagtt 480
tgtgaccctg tcaattcaag gcataatact tataacgggt ttctcgctga aaacatattc 540
tgcaaggctc cagaagtccg ctttgattgt cgtgaaaagg acgttgagaa actggtagag 600
gaactcgacg taaagccatt tgctgagatt attgataaac accacagtga aatttctggt 660
gttgtgatgg aaagcatcgt acagggcgct ggtggtctaa gaatgtatca tccatatttc 720
ctgaaacgtg tccgtgcact ctgcaacgac tttaatattt tgttgattct tgatgaagtt 780
gcagtggggc ttggaagaac gggtatgctg tttggatttg agcatgcagg gatagtgcct 840
gacatagtct gtctaggtaa gactttgacg gccggttatt tgacgttatc tgcaacagtt 900
accaccagag agattggtga tcagatttcc agtggacctg agggttgctt catgcacgga 960
caaacctata tggcaaatcc gttagcatgc gctgttgcaa gtgaaaactt gtcgatctta 1020
atggaaggta aatggaaatc tcaagttcgg caaatagaag tccagctgaa gaaagagctg 1080
gtgcccttac tggaacaccc catcgtggct gatgtaagaa ttctaggggc tattggtgtt 1140
gtcgaggtta caaaacgtgt gaacgtcgaa gtcttacaag agaaatttat taaggcaggt 1200
gcgtggatta gacctttcgg taacataatc tacattctac ctccatacat aattacttcc 1260
gaggagttga ccgtcttaac agaagctatc agatctgttc tagactttat ttag 1314

<210> 10
<211> 346
<212> PRT
<213> artificial

<220>
<223> BIO1 protein

<400> 10

Met Asn Thr Lys Ser Leu Asp Phe Tyr Glu Pro Leu Glu Ile Asp Gly
1               5                   10                  15

Gln Lys Tyr Ile Lys Met Thr Lys Lys Glu Asp Leu Gly Val Tyr Glu
            20                  25                  30

Ala Gly Val Thr Gln Glu Ala Phe Thr Ala Lys Asp Lys Tyr Asp Tyr
        35                  40                  45

Lys Gly Ile Val Glu Asn Leu Glu Arg Tyr Gly Leu Cys Ile Val Pro
    50                  55                  60

Ser Phe Ile Glu Pro Ser Lys Cys Asp Gln Ile Leu Glu Glu Leu Asp
65                  70                  75                  80

Pro His Phe Tyr Arg His Asp Ala Trp Gln Gly Ser Pro Phe Pro Lys
                85                  90                  95

Glu Thr Thr Val Val Thr Arg Ala Val Leu His Ser Pro Thr Val Leu
            100                 105                 110

Lys Glu Val Val Ser Asp Arg Leu Phe Cys Asp Met Ala Asn His Phe
        115                 120                 125

Leu Asn Glu Glu Asn Tyr Phe Leu Thr Gly Asn Val Ile Arg Lys Cys
    130                 135                 140

Ser Ser Gly Ile Gln Leu Asn Ser Gly Ile Val Tyr Lys Val Gly Ala
145                 150                 155                 160

Gly Ala Gly Asp Gln Gly Tyr His Arg Glu Asp His Ile His His Thr
                165                 170                 175

Ile His Gln Ala Arg Asp Ser Phe Gln Tyr Gly Lys Glu Thr Leu Leu
            180                 185                 190

Gly Val Gly Val Ala Phe Thr Asp Met Asn Lys Ala Asn Gly Ala Thr
        195                 200                 205

Arg Val Ile Ile Gly Ser His Leu Trp Gly Pro His Asp Ser Cys Gly
    210                 215                 220

Lys Phe Asp Lys Arg Met Glu Phe His Val Asn Ala Ala Lys Gly Asp
225                 230                 235                 240

Ala Val Leu Phe Leu Gly Ser Leu Tyr His Ala Ala Ser Ala Asn His
                245                 250                 255

Thr Leu Glu Asp Arg Ile Ala Gly Tyr Phe Phe Met Ser Gln Gly Tyr
            260                 265                 270

Leu Lys Gln Glu Glu Asn Leu His Phe Gly Thr Asp Pro Glu Phe Phe
        275                 280                 285

Lys Asp Met Pro Leu Glu Thr Leu Lys Leu Leu Gly Leu Thr Thr Ser
    290                 295                 300

Glu Pro Tyr Cys Gly His Ile Asp Tyr Lys Ser Pro Gly His Ile Ala
305                 310                 315                 320

Asn Pro Asn Leu Phe Glu Asn Glu Val Glu Lys Gly Tyr Tyr Gly Glu
                325                 330                 335

Thr Ile Lys Ile Ile Tyr Asp Asp Lys Glu
            340                 345

<210> 11
<211> 396
<212> PRT
<213> artificial

<220>

<223>  BIO2 protein

<400>  11

Met Asn Arg Gln Ser Leu Met Ala Gly Arg Arg Leu Phe Ser Lys Val
1               5                   10                  15

Arg Val Val Arg Lys Pro Ser Thr Leu Ala Asn Glu Leu Gly Ser Leu
            20                  25                  30

Tyr Glu His Asn Gln Pro Ile Phe Glu Pro Pro Ser Pro Glu Lys Lys
        35                  40                  45

Val Ser Ala Leu Asp Tyr Ala Phe Ser Leu Lys Ser Pro Val Ser Thr
    50                  55                  60

Trp Thr Lys Glu Gln Leu Arg Glu Ile Tyr Asn Thr Pro Leu Met Glu
65                  70                  75                  80

Leu Val His Arg Ala Gln Ile Tyr His Arg Asn Phe His Asn Pro Ser
                85                  90                  95

Glu Val Gln Ile Cys Asn Leu Val Asn Ile Lys Ser Gly Gly Cys Thr
            100                 105                 110

Glu Asp Cys Lys Tyr Cys Ser Gln Ser Val Asn Asn Asp Thr Asn Leu
        115                 120                 125

Lys Ala Glu Lys Leu Met Thr Val Asp Glu Val Val Glu Gln Ala Lys
    130                 135                 140

Ile Ala Lys Ser Asn Gly Ala Thr Arg Val Cys Leu Gly Ala Ala Tyr
145                 150                 155                 160

Arg Thr Val Ser Thr Arg Lys Arg Ala Leu Glu Arg Ile Thr Thr Met
                165                 170                 175

Val Lys Glu Val Asn Lys Leu Gly Leu Glu Ser Cys Val Thr Leu Gly
            180                 185                 190

Met Ile Glu Glu Glu His Ala Arg Lys Leu Lys Asp Ala Gly Leu Thr
        195                 200                 205

Ala Tyr Asn His Asn Ile Asp Thr Ser Arg Glu His Tyr Pro Asn Ile
    210                 215                 220

Ile Thr Thr Arg Thr Tyr Asp Asp Arg Leu Lys Thr Ile Asp Asn Val
225                 230                 235                 240

Gln Lys Ala Gly Ile Ser Ala Cys Thr Gly Gly Ile Leu Gly Leu Gly
                245                 250                 255

Glu Thr Val Glu Asp His Ile Gly Phe Leu Tyr Thr Leu Cys Thr Met
            260                 265                 270

Lys Gln His Pro Glu Ser Leu Pro Ile Asn Arg Leu Val Pro Ile Lys
        275                 280                 285

Gly Thr Pro Ile Ala Asp Glu Leu Ala Arg Asp Asn Ser Lys Lys Leu
    290                 295                 300

Lys Phe Glu Ser Met Leu Arg Thr Ile Ala Thr Ala Arg Met Val Met
305                 310                 315                 320

Pro Thr Ser Ile Ile Arg Leu Ala Ala Gly Arg Tyr Thr Met Lys Glu
                325                 330                 335

Thr Glu Gln Phe Leu Cys Phe Met Ala Gly Cys Asn Ala Ile Phe Ser
            340                 345                 350

Gly Lys Lys Met Leu Thr Thr Met Cys Asn Gly Phe Asp Glu Asp Lys
        355                 360                 365

Glu Leu Leu Ala Lys Trp Gly Met Lys Pro Met Gln Ser Phe Ala Ser
    370                 375                 380

Lys Arg Pro Ser Ser Ile Val Glu Thr Cys His Ile
385                 390                 395

<210>  12
<211>  480
<212>  PRT
<213>  artificial

<220>
<223>  BIO3 protein


<220>
<221>  misc_feature
<222>  (229)..(229)
<223>  Xaa can be any naturally occurring amino acid

<220>
<221>  misc_feature
<222>  (384)..(384)
<223>  Xaa can be any naturally occurring amino acid

<400>  12

Met Ser Gln Glu Ile Ser Tyr Thr Pro Asp Val Ala Glu Leu Leu Asp
1               5                   10                  15

Phe Asp Lys Lys His Ile Trp His Pro Tyr Thr Ser Leu Ser Ser Pro
            20                  25                  30

Leu Asn Val Tyr Pro Val Lys Ser Ala His Gly Cys Lys Leu Val Leu
        35                  40                  45

Asp Thr Asp Ser Pro Val Asp Val Glu Val Ile Asp Ala Met Ser Ser
    50                  55                  60

Trp Trp Cys Val Ile His Gly Tyr Asn Asn Pro Glu Leu Asn Glu Ala
65                  70                  75                  80

Leu Thr Lys Gln Met Leu Lys Phe Ser His Val Leu Leu Gly Gly Phe
                85                  90                  95

Thr His Lys Gly Ala Val Asn Leu Val Gln Lys Leu Leu Lys Val Ile
            100                 105                 110

Asp Glu Pro Ser Leu Gln Tyr Cys Phe Leu Ala Asp Ser Gly Ser Val
        115                 120                 125

Ala Val Glu Val Ala Leu Lys Met Ala Leu Gln Ser Asn Met Ser Gly
    130                 135                 140

Glu Ala Thr Lys Asn Arg Thr Lys Phe Leu Thr Ile Lys Asn Gly Tyr
145                 150                 155                 160

His Gly Asp Thr Phe Gly Ala Met Ser Val Cys Asp Pro Glu Asn Ser
                165                 170                 175

Met His His Ile Tyr Asn Asp Arg Leu Ser Glu Asn Ile Phe Ala Gln
            180                 185                 190

Ala Pro Ser Ile Val Asp Gly Leu Pro Thr Ser Gln Asn Gly Phe Glu
        195                 200                 205

Asp His Trp Asn Ala Glu Glu Val Thr Asp Leu Lys Lys Gln Phe Glu
    210                 215                 220

Leu His Ser Asp Xaa Ile Cys Ala Val Ile Leu Glu Pro Ile Leu Gln
225                 230                 235                 240

Gly Ala Gly Gly Leu Arg Pro Tyr His Pro Gln Phe Leu Ile Glu Val
                245                 250                 255

Gln Lys Leu Cys Asn Gln Tyr Asp Val Leu Phe Ile Met Asp Glu Ile
            260                 265                 270

Ala Thr Gly Phe Gly Arg Thr Gly Glu Ile Phe Ala Phe Lys His Cys
        275                 280                 285

Gln Lys Tyr Gln Asp Gln Arg Gly Ile Ser Pro Ser Asp Gln Ile Lys
    290                 295                 300

Val Val Pro Asp Ile Leu Cys Val Gly Lys Gly Leu Thr Ser Gly Tyr
305                 310                 315                 320

Met Thr Met Ser Ala Val Val Val Asn Asp Lys Val Ala Ser Arg Ile
                325                 330                 335

Ser Ser Pro Asn Ser Pro Thr Gly Gly Cys Phe Met His Gly Pro Thr
            340                 345                 350

Phe Met Gly Asn Ala Leu Ala Cys Ser Val Ala Glu Lys Ser Met Asp
        355                 360                 365

Ile Leu Leu Arg Gly Glu Trp Arg Lys Gln Val Ser Ala Ile Glu Xaa
    370                 375                 380

Gln Ile Tyr Arg Glu Leu Tyr Gln Tyr Ile Lys Asn Pro Asp Asn Gly
385                 390                 395                 400

Leu Ile Gly Thr Val Val Lys Arg Val Ser Val Ile Gly Ala Val Gly
                405                 410                 415

Ile Val Glu Leu Tyr Lys Lys Thr Asp Pro Glu Trp Phe Gln Lys Lys
            420                 425                 430

Phe Ile Ser Lys Gly Val His Ile Arg Pro Phe Asn Cys Leu Cys Tyr
        435                 440                 445

Ile Met Pro Pro Tyr Val Ile Thr Thr Glu Glu Leu Thr Lys Val Asn
    450                 455                 460

Gln Val Leu Ile Glu Val Leu His Glu Trp Lys Ser His Ile Asn Gln
465                 470                 475                 480

<210> 13
<211> 237
<212> PRT
<213> artificial

<220>
<223> BIO4 protein

<400> 13

Met Asn Ser Lys Ser Gln Gln Gln Glu Gln Gln Pro Ile Val Phe Val
1               5                   10                  15

Thr Gly Thr Asp Thr Asp Val Gly Lys Thr Phe Val Ser Thr Leu Leu
            20                  25                  30

Val His Lys Trp Lys Ala Ala Tyr Trp Lys Pro Val Gln Thr Gly Ile
        35                  40                  45

Glu Ser Asp Gln Gly Asp Ser Glu Thr Leu Lys Asn Phe Lys Ile Ala
    50                  55                  60

Ala Ser Thr Trp Gln Pro Pro Ile Phe Thr Pro Thr Tyr Ala Leu Gln

65                  70                  75                  80

Lys Pro Leu Ser Pro Leu Gln Ala Met Glu Tyr Glu Pro Asn Val Asp
                85                  90                  95

Ile Arg Leu Leu Asp Phe Val Val Pro Glu Glu Trp Ser Ala Glu Asn
            100                 105                 110

Pro Leu Val Val Glu Gly Ala Gly Gly Val Cys Val Pro Ile Thr Arg
        115                 120                 125

Lys Leu Glu Ile Thr Thr Asp Leu Ile Lys His Leu Ile Glu Thr Ser
    130                 135                 140

Gly His Pro Val Tyr Val Val Val Val Ala Arg Ser Gly Leu Gly Thr
145                 150                 155                 160

Leu Asn His Thr Leu Leu Thr Trp Asn His Leu Cys Asp Asn Gly Leu
                165                 170                 175

Arg Ser His Leu Phe Gly Val Ile Leu Asn Gly Glu Pro Asn Glu Gly
            180                 185                 190

Asn Val Gln Ala Leu Lys Lys Phe Gly Val Asn Ile Met Ala Gln Val
        195                 200                 205

Ala Gln Cys Thr Thr Ala His Asp Gln Asp Met Ala Leu His Glu Leu
    210                 215                 220

Pro Ser Val Glu Ser Leu Met Thr Gln Gln Asp Val Glu
225                 230                 235


<210>  14
<211>  561
<212>  PRT
<213>  artificial

<220>
<223>  BIC5 protein

<400>  14

Met Asn Arg Val Gly Ala Val Phe Leu Phe Val Tyr Glu Arg Asn Phe
1               5                   10                  15

Phe Leu Ser Ile Val Pro Asp Arg His Arg Thr Glu Ile Arg Met Ser
            20                  25                  30

Ser Ser Glu Arg Ser Glu Val Lys Phe Asp Lys His Phe Asn Trp Trp
        35                  40                  45

Ser Leu Leu Gly Ile Ala Phe Ser Leu Ser Cys Ser Trp Val Gly Ile
    50                  55                  60

Ser Ala Ser Met Ala Val Gly Ile Ala Ser Gly Gly Pro Leu Leu Ile
65                  70                  75                  80

Ile Tyr Gly Leu Ile Ile Ala Ala Phe Phe Ser Leu Met Cys Gly Ile
                85                  90                  95

Ser Leu Gly Asp Phe Ala Ala Ile Leu Pro Asn Ser Ser Gly Gly Ser
            100                 105                 110

Phe Trp Val Leu Lys Met Leu Glu Gln Glu Ser Val Thr Leu Lys Thr
        115                 120                 125

Pro Glu Tyr Glu Asp Pro Ser Asp Asp Asp Glu Glu Val Phe Leu Glu
    130                 135                 140

Asn Tyr Cys Gln Thr Phe Asn Val Glu Val Ser Ser Lys Phe Gln Lys
145                 150                 155                 160

Val Ser Ser Met Val Val Gly Leu Leu Asn Tyr Phe Gly Ala Ile Phe
                165                 170                 175

Thr Thr Ala Ser Ile Cys Ser Ser Leu Ser Met Ser Cys Ile Gly Ile
            180                 185                 190

His Lys Leu Leu His Pro Asp Tyr Glu Leu Lys His Trp His Val Phe
        195                 200                 205

Val Gly Tyr Glu Cys Ile Asn Ala Val Leu Thr Leu Phe Asn Ile Tyr
    210                 215                 220

Ser Thr Pro Leu Pro Tyr Ile Ser Gln Phe Gly Leu Tyr Thr Ser Leu
225                 230                 235                 240

Leu Ser Phe Ala Met Thr Phe Ile Ile Cys Ile Val Ser Arg Ser Asp
                245                 250                 255

Asn Thr Val Asp Pro Trp Pro Lys Ala Ser Asn Ile Phe Gly Ser Phe
            260                 265                 270

Asp Asn Gln Thr Gly Trp Asn Ser Ser Gly Met Ala Phe Val Val Gly
        275                 280                 285

Leu Val Asn Pro Ile Trp Ala Phe Val Gly Ile Asp Ser Ala Thr His
    290                 295                 300

Met Ile Asp Glu Val Gly Tyr Ser Lys Ser Arg Phe Leu Val Pro Lys
305                 310                 315                 320

Val Ile Ile Thr Thr Ile Ile Val Gly Phe Val Thr Ser Phe Ile Tyr
                325                 330                 335

Cys Val Gly Leu Phe Phe Cys Ile Thr Asp Gln Thr Ala Val Val Glu
            340                 345                 350

Ser Ile Leu Pro Ile Val Glu Ile Phe Tyr Gln Ala Thr Gly Asn Arg
        355                 360                 365

Asn Leu Ser Val Phe Leu Gln Cys Met Cys Ile Thr Thr Gly Phe Val
    370                 375                 380

Ser Gly Ile Ala Ser Gly Thr Trp Gln Ser Arg Ile Leu Gln Ser Phe
385                 390                 395                 400

Gly Lys Ser Tyr Ala Pro Phe Tyr Lys Glu Gly Ser Leu Gly Asn Lys
                405                 410                 415

Ser Leu Lys Lys Leu Ala Val Leu Thr Pro Gly Phe Lys Ser Pro Leu
            420                 425                 430

Tyr Ala His Phe Leu Ser Gln Ile Cys Val Thr Ile Ile Gly Cys Ile
        435                 440                 445

Phe Met Gly Ser Ser Thr Ala Phe Asn Ala Ile Ile Thr Ala Cys Ile
    450                 455                 460

Thr Leu Leu Leu Met Ser Tyr Ala Val Pro Ser Phe Ile Phe Leu Phe
465                 470                 475                 480

Val Ile Lys Lys Glu Lys Phe Ile His Arg Ile Glu Ser Asp Val Asn
                485                 490                 495

Cys Val Ser Arg Pro Asn Arg Arg Arg Met Ser Met Ile Pro His Ile
            500                 505                 510

Ile Cys Ile Leu Trp Thr Leu Phe Cys Leu Val Phe Leu Ser Phe Pro
        515                 520                 525

Tyr Thr Leu Pro Val Thr Ala Gly Asn Met Asn Tyr Thr Ser Val Val
    530                 535                 540

Tyr Ala Val Val Phe Cys Ile Ile Ser Ile Val Val Phe Pro Thr Cys
545                 550                 555                 560

Ile


<210> 15
<211> 437
<212> PRT
<213> artificial

<220>
<223> BIO6 protein

<400> 15

Met Leu Glu His Gln Leu Thr Gln Glu Asp Leu Glu Phe Asp Lys Lys
1               5                   10                  15

His Ile Trp His Pro Tyr Thr Ser Ile Thr Thr Pro Leu Lys Val Tyr
            20                  25                  30

Pro Ile Thr Lys Ala Glu Gly Ser Tyr Leu Tyr Leu Asp Asn Gly Thr
        35                  40                  45

Lys Val Val Asp Gly Met Ala Ser Trp Trp Cys Val Gln Gln Gly Tyr
    50                  55                  60

Asn Asn His Arg Leu Asn Ala Ala Ala Ile Ser Gln Ile Asn Lys Met
65                  70                  75                  80

Ser His Val Met Phe Gly Gly Ile Thr His Lys Ala Gly Ile Asp Phe
                85                  90                  95

Cys Lys Lys Leu Leu Ala Leu Leu Pro Asp Thr Leu Glu Cys Ala Leu
            100                 105                 110

Leu Ala Asp Ser Gly Ser Ile Ser Val Asp Ile Ala Met Lys Met Ala
        115                 120                 125

Leu Arg Tyr His His Ser Leu Gly Asn Thr Thr Lys Lys Arg Phe Leu
    130                 135                 140

Thr Ile Lys Lys Gly Tyr His Gly Asp Ala Phe Gly Ala Val Ser Val
145                 150                 155                 160

Cys Asp Pro Val Asn Ser Arg His Asn Thr Tyr Asn Gly Phe Leu Ala
                165                 170                 175

Glu Asn Ile Phe Cys Lys Ala Pro Glu Val Arg Phe Asp Cys Arg Glu
            180                 185                 190

Lys Asp Val Glu Lys Leu Val Glu Glu Leu Asp Val Lys Pro Phe Ala
        195                 200                 205

Glu Ile Ile Asp Lys His His Ser Glu Ile Ser Gly Val Val Met Glu
    210                 215                 220

Ser Ile Val Gln Gly Ala Gly Gly Leu Arg Met Tyr His Pro Tyr Phe
225                 230                 235                 240

Leu Lys Arg Val Arg Ala Leu Cys Asn Asp Phe Asn Ile Leu Leu Ile
                245                 250                 255

Leu Asp Glu Val Ala Val Gly Leu Gly Arg Thr Gly Met Leu Phe Gly

            260                 265                 270

Phe Glu His Ala Gly Ile Val Pro Asp Ile Val Cys Leu Gly Lys Thr
        275                 280                 285

Leu Thr Ala Gly Tyr Leu Thr Leu Ser Ala Thr Val Thr Thr Arg Glu
    290                 295                 300

Ile Gly Asp Gln Ile Ser Ser Gly Pro Glu Gly Cys Phe Met His Gly
305                 310                 315                 320

Gln Thr Tyr Met Ala Asn Pro Leu Ala Cys Ala Val Ala Ser Glu Asn
                325                 330                 335

Leu Ser Ile Leu Met Glu Gly Lys Trp Lys Ser Gln Val Arg Gln Ile
            340                 345                 350

Glu Val Gln Leu Lys Lys Glu Leu Val Pro Leu Leu Glu His Pro Ile
        355                 360                 365

Val Ala Asp Val Arg Ile Leu Gly Ala Ile Gly Val Val Glu Val Thr
    370                 375                 380

Lys Arg Val Asn Val Glu Val Leu Gln Glu Lys Phe Ile Lys Ala Gly
385                 390                 395                 400

Ala Trp Ile Arg Pro Phe Gly Asn Ile Ile Tyr Ile Leu Pro Pro Tyr
                405                 410                 415

Ile Ile Thr Ser Glu Glu Leu Thr Val Leu Thr Glu Ala Ile Arg Ser
            420                 425                 430

Val Leu Asp Phe Ile
        435

## Description

The present invention relates to a biotin prototrophic yeast, which can be used as a host cell for expression of various recombinant proteins, by cultivation in biotin-deficient medium.

Yeasts are eukaryotic microorganisms classified in the kingdom Fungi, with about 1,500 species currently described. Most reproduce asexually by budding, although a few do so by binary fission. Yeasts are unicellular, although some species with yeast forms may become multicellular through the formation of a string of connected budding cells known as pseudohyphae, or false hyphae as seen in most molds.

The most common species of yeast is Saccharomyces cerevisiae. Other species of yeast, such as Candida albicans, are opportunistic pathogens and can cause infection in humans. Saccharomycetes is a class in the kingdom of fungi. It contains the order Saccharomycetales, the budding yeasts, sometimes called Hemiascomycetes. Saccharomycetales is an order in the kingdom of fungi that comprises the budding yeasts.

The yeast species Saccharomyces cerevisiae is a model organism in cell biology research, and is one of the most thoroughly researched eukaryotic microorganisms. It reproduces by a division process known as budding.

The development of the recombinant DNA techniques has permitted the use of several microorganisms as host for the expression of heterologous proteins with pharmaceutical and industrial application. The yeast Saccharomyces cerevisiae is among the eukaryotic microorganisms most frequently used in the current biotechnology. However, yeasts from the genera Pichia, Hansenula, Komagataella, Yarrowia, Candida and Kluyveromyces, have also been used efficiently as expression systems.

Methylotrophic yeasts, represented by the genera Hansenula, Pichia, Komagataella, Candida and Torulopsis, can utilize reduced one-carbon compounds, such as methanol or methane, as the carbon source for their growth.

Pichia pastoris, a methylotrophic yeast, is frequently used as an expression system for the production of recombinant proteins, and more recently also for the production of small metabolites (Marx et al. Microb Cell Fact 7:23 (2008)). Pichia has a high growth rate and is able to grow on a simple, inexpensive medium. Pichia can grow in either shake flasks or a fermenter, which makes it suitable for both small and large scale production.

Pichia pastoris has recently been reclassified into a new genus, Komagataella, and been separated into three new species: Komagataella pastoris, K. phaffii, and K. pseudopastoris (Kurtzman CP. Int J Syst Evol Microbiol 55, 973-976. (2005)). Therefore, Pichia pastoris is a synonym for all three species, K. pastoris, K. phaffii and K. pseudopastoris. In accordance with previous literature, Pichia pastoris is used throughout this text, implicitely meaning any of the Komagataella species. Similarly, Hansenula polymorpha and Pichia angusta are synonyms.

Yeasts are attractive hosts for the production of recombinant proteins and small metabolites. Apart from easy genetic manipulation, one of their major advantages is their high growth rates at cheap minimal media. Ideally, the cultivation media should consist only of an utilizable carbon source, salts containing the macro elements (N, O, S, Mg, K) and trace elements. However, due to the auxotrophy of many yeast strains, vitamin solutions routinely have to be added, preventing the use of a truly mineral medium.

The use of complex media components, such as yeast extract or peptone, which would overcome such auxotrophies, is critical as there is a big lot-to-lot variability (Zhang et al. Biotechnol Bioeng 82(6):640-52 (2003)). This is not only in contradiction to the regulatory requirements; it also needs extensive product purification in down-stream processing.

In the "mineral medium" suggested by Theobald et al. (Anal Biochem 214(1):31-7. (1993)), which has been used for cultivations of various yeasts, e. g. Saccharomyces cerevisiae, S. bayanum, Kluyveromyces marxianus, K. lactis, a vitamin cocktail containing biotin, calcium pantothenate, inositol, pyridoxine HCl and thiamine HCl is usually provided, regardless of the requirements of the respective yeast strains.

Biotin plays an essential role as cofactor for biotin-dependent carboxylases carrying out carboxylation and decarboxylation reactions involved in important metabolic pathways such as fatty acid biosynthesis, gluconeogenesis and amino acid metabolism (Streit W, Entcheva P. Appl Microbiol Biotechnol 61(1):21-31 (2003)). Most microorganisms and plants have the ability to synthesize biotin de *novo,* whereas animals rely on the dietary uptake of the vitamin. The biotin biosynthesis pathway has been extensively studied in bacteria, especially Escherichia coli and Bacillus subtilis, and more recently also the pathways in rhizobia (Guillén-Navarro et al. FEMS Microbiol Lett 246(2):159-65 (2005)) and plants, as Arabidopsis thaliana (Muralla et al. Plant Physiol 146(1):60-73 (2008); Pinon et al. Plant Physiol 139(4):1666-76 (2005)) were elucidated.

Starting from the precursors pimeloyl-CoA and alanine, four conserved reactions lead to the formation of biotin: In the first step, 7-keto-8-aminopelargonic acid (KAPA) is formed by KAPA synthetase, which is then further converted to 7,8-diaminopelargonic acid (DAPA) by DAPA aminotransferase, using S-adenosylmethionine (SAM) as amino-donor. Dethiobiotin synthetase catalyzes the ATP-dependent carboxylation reaction leading to dethiobiotin (DTB), which is finally synthesized to biotin in a SAM-dependent reaction catalyzed by biotin synthetase. The first steps of the pathway leading to pimeloyl-CoA are not conserved among the species. Gram-positive bacteria, e.g. Bacillus ssp., and plants use pimelic acid as precursor (Baldet et al. Eur J Biochem 217(1):479-85 (1993)) while gram-negative bacteria most probably utilize fatty acid metabolites.

In contrast to the majority of microorganisms, some yeast species - especially members of the order of Saccharomycetales - have lost the ability to synthesize biotin themselves. The inability of an organism to synthesize a particular compound required for its growth is also called auxotrophy. Despite of their biotin auxotrophy, the yeast species still possess various genes of the biotin biosynthesis pathway. All of the yeast strains sequenced up to date have a copy of the BIO2 gene coding for biotin synthetase. In S. cerevisiae strains, three genes encoding DAPA aminotransferase (BIO3), DTB synthetase (BIO4) and a unique KAPA transporter (BIO5) are localized in a cluster.

The prior art literature in general describes the biotin biosynthesis pathway and functions of the individual genes.

Phalip et al. (Gene 232(1):43-51 (1999)) engineered mutants of S. cerevisiae by introducing BIO3 and BIO4 to analyze their function.

In the course of sequencing the genome of sake yeast strains, which were prototrophic for biotin, a yeast homolog of the KAPA synthetase gene (BIO6) was identified (Wu et al. Appl Environ Microbiol 71(11):6845-55 (2005)). Wu et al. analyzed BIO3 and BIO6 disrupted mutants for their ability to grow in a biotin-deficient medium, supplemented with key precursors of the biotin biosynthesis pathway as e.g. KAPA.

Recently, the missing step in the biotin synthesis pathway in yeasts was elucidated, as the protein encoded by a gene adjacent to BIO6 was found having the function of a pimeloyl-CoA synthetase (PCAS, BIO1). The latter two genes were described to be acquired through gene duplication and horizontal gene transfer from bacteria in an ancestor of the yeast (Hall C and Dietrich F. Genetics 177(4):2293-307 (2007)). Hall and Dietrich introduced BIO1 and BIO6 genes into the biotin auxotrophic strain S288c of S. cerevisiae, resulting in cell growth in biotin-lacking media.

Hong et al. (Appl Microbiol Biotechnol. 71:211-221 (2006)) disclosed biotin production using Candida utilis, by cloning the BIO2 gene into the C. utilis chromosome, to increase the biotin yield of 1 00-fold above the endogenous level.

US 7,033,814B2 describes a method of preparing yeast with improved biotin productivity by introducing a Candida utilis BIO2 or a Saccharomyces cerevisiae BIO2 gene, for producing biotin in E. coli or yeast expression systems.

GB 2216530A describes plasmids containing one or more genes of the biotin biosynthesis pathway, derived from E. coli, which are capable of replication and expression in another organism than E. coli. Thereby the yield of biotin from an organism which exports biotin may be improved or intracellular levels increased.

The cultivation of yeast, e.g. Pichia pastoris, usually requires addition of high dosage of biotin, e.g. at least 2 x 10⁻⁵ g/L, a minimum concentration of 4 x 10⁻⁴ g/L is commonly used. As biotin is the only non-salt media component used during P. pastoris fermentation, apart from the carbon source, nonconformities during protein production processes are usually attributed to poor quality of the added biotin, e.g. too old preparations of the compound, not completely dissolved solutions, and failed sterile filtration. Additionally, biotin free media are cheaper, have a longer shelf life and do not require cooling for storage.

In order to circumvent quality issues of the biotin containing media and consequently dismissed productions runs, it is the object of the present invention to provide a prototrophic yeast production host, for use in the production of recombinant gene products.

The object is solved by the host cell line according to the invention, which is a host cell line of a biotin prototrophic yeast comprising a BIO1 gene and a xenogene. Thus, the biotin prototrophic organism would be able to grow in media of low biotin concentrations.

The host cell line according to the invention preferably is provided as a production cell line, ready to use for cultivating in a bioreactor, which is preferably at least a 1 L bioreactor, more preferred at least 2L or at least 5L, e.g. for pilot scale production, up to industrial scale production, which refers to bioreactor volumes of at least 50L, more preferably at least 100L.

The host cell according to the invention preferably is a budding yeast, such as a yeast of a Saccharomycetales, more preferably a yeast is selected from the group consisting of Pichia, Saccharomyces, Kluyveromyces, Hansenula, Yarrowia and Komagataella, more preferably selected from the group consisting of Pichia pastoris, Saccharomyces cerevisiae and Hansenula polymorpha.

The host cell line according to the invention preferably further comprises at least one of a BIO gene encoding a biotin biosynthetic enzyme, such as those selected from the group consisting of BIO2, BIO3, BIO4, BIO5, BIO6.

According to a preferred embodiment at least one of BIO1 or other BIO genes is heterologous to the organism.

The invention further refers to a Pichia pastoris comprising a BIO1 gene, and the respective cell line, most preferably a production cell line, such as a master cell line.

A preferred host cell line according to the invention is a one of Pichia pastoris comprising a BIO1, BIO2, BIO3, BIO4 and BIO6 gene, wherein at least one of the BIO genes is of Saccharomyces cerevisiae origin.

According to the invention it is the first time possible to provide a biotin prototrophic Pichia pastoris. The biotin prototrophic Pichia pastoris according to the invention preferably comprises a xenogene and/or a BIO1 gene.

The invention further refers to a method of producing a host cell line of a biotin prototrophic yeast comprising a BIO1 gene and a xenogene, comprising the steps of:
a) providing a yeast host cell,
b) providing a BIO1 gene and a xenogene,
c) introducing said genes into said host cell, and
d) cultivating said host cell in a biotin-deficient medium to obtain a host cell line.

In a preferred method according to the invention said genes are comprised in separate vectors.

Alternatively, in another preferred embodiment of the invention said genes are comprised in a single vector.

According to the invention there is further provided a vector for use in a method according to the invention, comprising a BIO1 gene and/or a gene coding for a xenogenic polypeptide.

According to the invention it is further preferred to produce a product, such as a xenogenic polypeptide or a metabolite of a host cell, in a yeast host cell line obtained by a method according to the invention, characterized in that said xenogene is expressed, and said product is isolated, preferably as a functional biomolecule in the purified form.

Preferably said product is selected from the group consisting of serum proteins, such as immunoglobulins or serum albumin, enzymes, hormones, signaling molecules, matrix proteins, fragments or derivatives thereof.

According to another preferred embodiment the xenogene encodes a polypeptide that is bringing about a metabolite. Thus, the product of the recombinant host cell line is preferably a progenitor or reactant to produce a metabolite of said host cell. The preferred reactant is either a substrate, enzyme or cofactor, capable of a metabolizing reaction to bring about a commercially valuable organic molecule, like functional biomolecules, such as organic acids, alcohols or vitamins, in particular riboflavin.

The term "host cell" or "hosts cell line" refers to a microorganism, used for expression of a recombinant gene to produce polypeptides or metabolites mediated by such polypeptides. A host cell clone of cultivated host cells that have proliferated is commonly understood to be a host cell line. A production host cell line is commonly understood to be a cell line ready-to-use for cultivation in a bioreactor to obtain the gene product in a production method.

The term "biotin prototrophy" means the ability of a microorganism to produce biotin or its precursors during its cultivation to support cell proliferation and growth.

The term "BIO gene" refers to the gene coding for an enzyme necessary to process biotin precursors or vitamers, such as Pimeloyl-CoA synthase, biotin synthetase, DAPA aminotransferase, dethiobiotin synthetase, KAPA transferase and KAPA synthase, as well as homologues thereof to produce functional equivalents. The term BIO gene in particular refers to the genes originating either from eukaryotic organisms, like yeast cells, or to those coding for functional equivalents thereof, such as those originating from prokaryotic organisms. Thus, the term "BIO1 gene" also refers to e.g. functional equivalents of bioC and bioH of E. coli, "BIO2 gene" to bioB, "BIO3 gene" to bioA, "BIO4 gene" to bioD and "BIO6 gene" to bioF. BIO genes are illustrated by Sequ. ID No. 4-9.

The term "xenogene" refers to a gene coding for a polypeptide foreign to an organism, which gene is not comprised in the genome of a wild-type organism. Preferably the xenogene is derived from a mammal, or fish, or insect cell, or plant, or fungal cell or prokaryotic origin, or homologues thereof. Preferably the xenogene is not derived from a wild-type yeast cell. The term encompasses nucleic acid sequences, either DNA or RNA.

The term "foreign" refers to substances that are not needed for cell growth. Thus, substances that supply an autotrophic requirement, such as those encoded by a BIO gene, are not encompassed by such term. A BIO gene, either heterologous or homologous, is understood to be a gene coding for an endogenous protein necessary for the yeast cell growth, and thus is not foreign to the yeast cell. In the context of expressing a foreign polypeptide, it is understood that the term does not refer to a selection marker only. Foreign polypeptides usually are encoded by recombinant hosts.

The term "heterologous gene" refers to a gene, which does not exist in the host, or a partial DNA thereof. The term also includes nucleic acid sequences, either DNA or RNA, encoding a gene product, such as the BIO gene.

The expression "heterologous protein" is intended to indicate a protein or polypeptide, which is not produced by the host organism in nature.

The term "xenogenic polypeptide" refers to a polypeptide foreign to the host organism, which expresses or produces said polypeptide. Usually, the xenogenic polypeptide is from a different species. For examples, a mammalian or bacterial protein is considered to be a polypeptide, which is xenogenic to a yeast host cell.

The term "homologue" and "homology" with regard to polypeptides or proteins indicates that polypeptides have the same or conserved residues at a corresponding position in their primary, secondary or tertiary structure. The term also extends to two or more nucleotide sequences encoding the homologous polypeptides. In particular, homologous compounds usually have at least about 50% amino acid sequence identity with regard to a full-length native sequence or any fragment thereof. Preferably, a homologous compound will have at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native compound, or any other specifically defined fragment of a full-length compound.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The term "derivative" encompasses any combination of one or more compounds, e.g. polypeptides or proteins, and / or a fusion compound, in which any part of the compound may be fused at any position to one or more other polypeptides. A derivative may also be obtained by association or binding to other substances by various chemical techniques such as covalent coupling, electrostatic interaction, di-sulphide bonding etc. The other substances bound to the compound may be lipids, carbohydrates, nucleic acids, organic and inorganic molecules or any combination thereof (e.g. PEG, prodrugs or drugs). A derivative would also comprise a homologous compound, also potentially containing non-natural or chemically modified amino acids, and fragments of compounds. It is understood that the term BIO gene or xenogene, or their expression products, also refers to derivatives thereof, which may have similar or improved functions.

"Expression vectors" as used herein are defined as DNA sequences that are required for the transcription of cloned recombinant nucleotide sequences, i.e. of recombinant genes and the translation of their mRNA in a suitable host organism. Such expression vectors usually comprise an origin for autonomous replication in the host cells, selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together.

The terms "plasmid" and "vector" as used herein include autonomously replicating nucleotide sequences as well as genome integrating nucleotide sequences.

The term "polypeptide" refers to a protein or peptide that contains two ore more amino acids, typically at least 3, preferably at least 20, more preferred at least 30, more preferred at least 50 amino acids. The term also refers to higher molecular weight polypeptides, such as proteins.

The term "gene product" or "product of a gene" is the biochemical material, either RNA or protein, resulting from expression of a gene, such as a xenogene.

The host cell genome according to the invention comprises at least the BIO1 gene and a xenogene, to provide for the biotin prototrophy of the host expressing the xenogenic polypeptide. Thus, biotin auxotrophic yeasts, such as P. pastoris were engineered to become a biotin prototrophic yeast, that surprisingly was not only able to grow without adding exogenous biotin, but could also provide for recombinant protein production with high yields. The employed methods according the present invention can be easily adapted to other biotin auxotrophic yeasts used in biotechnology.

The yeast organism used according to the invention may be any suitable yeast organism which, on cultivation, produces large amounts of the heterologous protein or polypeptide in question. Examples of suitable yeast organisms may be strains selected from the yeast species Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans.

The most preferred yeast host cells are derived from a methylotrophic yeast, such as from Pichia or Komagataella, e.g. Pichia pastoris, or Komoagataella pastoris, or K. phaffii, or K. pseudopastoris.

The BIO1 gene contained in the host cell according to the invention may be a heterologous gene, but also a native gene, thus may be contained in the wild-type expression host as such. To become biotin prototrophic, the host cell according to the invention may need any of the other BIO genes as well, which may also be either heterologous or homologous. It is preferred that at least one of the BIO1 or other BIO genes is a heterologous gene.

The origin of such heterologous BIO gene is preferably from a plant, insect, fungal or bacterial species, preferably from a yeast, more preferably from a yeast strain different from the host yeast strain. In particular those BIO genes are preferred that are derived from Saccharomyces cerevisiae or E. coli. According to a preferred process an appropriate donor cell is derived from biotin prototrophic organisms.

A preferred Pichia host cell, such as a P. pastoris host cell, contains the heterologous BIO1, BIO6, BIO3 and BIO4 genes, which are preferably derived from a biotin prototrophic S. cerevisiae strain, e.g. from S. cerevisiae/paradoxus A364A (CBS 7302) or from a biotin auxotrophic S. cerevisiae strain, such as S. cerevisiae Meyen ex E.C. Hansen var. cerevisiae HA232 (CBS 1782; CBS Fungal Biodiversity Centre, Utrecht, The Netherlands).

According to another preferred embodiment the host cell is a Saccharomyces host cell, such as S. cerevisiae, containing heterologous BIO1 and BIO6 genes, preferably from S. cerevisiae/paradoxus A364A.

According to the invention it is preferred to provide a P. pastoris host comprising the BIO1 gene, and optionally at least one of the BIO3, BIO4 and BIO6 gene, which are preferably heterologous. The preferred host cell further contains an endogenous BIO2 gene. The P. pastoris strain according to the invention would be able to grow in a biotin deficient medium and advanteously used as an expression host cell line, that can be recombined with any heterologous gene.

The xenogenes preferably incorporated into the host cell according to the invention code for a polypeptide, such as polypeptides or proteins, preferably derived from eukaryotic organisms, like mammals, or fish, insects, plants, or fungal cells, but also from prokaryotic organisms, such as bacteria. Such polypeptide or proteins include, but are not limited to, those having use in the medical or scientific research fields.

The expression products of such xenogenes are preferably heterologous polypeptides or proteins, which may advantageously be produced in a eukaryotic cell, preferably a yeast cell, preferably as secreted proteins. Examples of preferably produced proteins are immunoglobulins, aprotinin, tissue factor pathway inhibitor or other protease inhibitors, and insulin or insulin precursors, insulin analogues, growth hormones, interleukins, tissue plasminogen activator, transforming growth factor a or b, glucagon, glucagon-like peptide 1 (GLP-1), glucagon-like peptide 2 (GLP-2), GRPP, Factor VII, Factor VIII, Factor XIII, platelet-derived growth factor1, serum albumin, enzymes, such as lipases or proteases, or a functional analogue of any one of these proteins. In the present context, the term "functional analogue" is meant to indicate a polypeptide with a similar function as the native protein. The polypeptide may be structurally similar to the native protein and may be derived from the native protein by addition of one or more amino acids to either or both the C- and N-terminal end or the side-chain of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

Xenogenes can also encode for substrates, enzymes, inhibitors or cofactors that provide for biochemical reactions in the host cell, with the aim to obtain the product of said biochemical reaction or a cascade of several reactions.

Examplary products can be vitamins, such as riboflavin, organic acids, and alcohols.

The DNA construct of the invention encoding the polypeptide of the invention may be prepared synthetically by established standard methods, e.g. the phosphoramidite method.

The DNA construct of the invention may also be of genomic or cDNA origin, for instance obtained by preparing a genomic or cDNA library and screening for DNA sequences coding for all or part of the polypeptide of the invention by hybridization using synthetic oligonucleotide probes in accordance with standard techniques (Sambrook et al., Molecular Cloning: A Laboratorv Manual, Cold Spring Harbor, 1989).

Finally, the DNA construct may be of mixed synthetic and genomic, mixed synthetic and cDNA or mixed genomic and cDNA origin prepared by annealing fragments of synthetic, genomic or cDNA origin, as appropriate, the fragments corresponding to various parts of the entire DNA construct, in accordance with standard techniques.

According to the present invention, a heterologous gene and/or a xenogene is ligated into a vector and a single vector is constructed, carrying the genes, or separate vectors to carry the heterologous genes or the xenogene. These genes can be stably integrated into the host cell genome by transforming the host cell using such vectors. The polypeptides encoded by the genes can be produced using the recombinant host cell line by culturing a transformant thus obtained in an appropriate medium, isolating the expressed product of the xenogene from the culture, and purifying it by a method appropriate for the expressed product.

Heterologous genes or xenogenes can be modified to be highly expressed in the yeast host cell according to the invention. The genes can be modified to enable high level expression in the yeast host cell according to the invention by optimizing the gene sequence to correlate with those codons most frequently used in the yeast. For example, the gene sequence can be optimized according to the codons used with genes which are highly expressed in the host.

The recombinant expression vector may be any vector which is capable of replicating in or integrating into the genome of yeast organisms, also called yeast vector, which carries a DNA construct according to the invention.

Preferably, the yeast expression vector of the invention is for expression in a yeast selected from the group consisting of methylotrophic yeasts represented by the genera Hansenula, Pichia, Candida and Torulopsis.

The procedures used to ligate the DNA sequences coding for the polypeptide of the invention, the promoter and the terminator, respectively, and to insert them into suitable yeast vectors containing the information necessary for integration or yeast replication, are well known to persons skilled in the art, e.g. described by J. Sambrook et al., "Molecular Cloning 2nd ed.", Cold Spring Harbor Laboratory Press (1989 ).

The foreign gene encoding the above-mentioned heterologous compounds or polypeptides can be inserted into the vector so as to be under the control of the promoter. In the present invention, it is preferred to use a pPICZ, pGAPZ, pPIC9, pPICZalfa, pGAPZalfa, pPIC9K, pGAPHis or pPUZZLE-derived plasmids as the vector.

It will be understood that the vector, which uses the BIO1 gene and/or the xenogene as an integration target, may be constructed either by first preparing a DNA construct containing the entire DNA sequence coding for the BIO gene and/or the xenogene and subsequently inserting this fragment into a suitable expression vector, or by sequentially inserting DNA fragments containing genetic information for the individual elements, such as the signal, leader or heterologous protein, followed by ligation.

Also multicloning vectors, which are vectors having a multicloning site, can be used according to the invention, wherein a desired heterologous gene can be incorporated at a multicloning site to provide an expression vector. Expression vectors are vectors carrying a heterologous gene and used for expression of a heterologous compound encoded by such gene. In expression vectors, the promoter is placed upstream of the gene of the heterologous compound and regulates the expression of the gene. In the case of multicloning vectors, because the gene of the heterologous compound is introduced at the multicloning site, the promoter is placed upstream of the multicloning site.

Transformants according to the present invention can be obtained by introducing a vector DNA, e.g. plasmid DNA, into a host such as P. pastoris and selecting transformants which grow in a biotin deficient medium and/or which express the compound encoded by the xenogene.

Host cells are treated to enable them to incorporate foreign DNAs by methods conventionally used for transformation of yeast cells, such as the electric pulse method, the protoplast method, the lithium acetate method, and modified methods thereof.

Examples of the host to be transformed with the vector according to the present invention include yeasts such as P. pastoris. Examples of P. pastoris strains include CBS 704 (=NRRL Y-1603 = DSMZ 70382), CBS 2612 (=NRRL Y-7556), CBS 7435 (=NRRL Y-11430), CBS 9173-9189, and DSMZ 70877 (German Collection of Microorganisms and Cell Cultures), examples of S. cerevisiae strains include W303, CEN.PK and the BY-series (EUROSCARF collection).

All of the strains described above have been successfully used to produce transformants and express heterologous genes. P. pastoris is preferably transformed by using an expression vector by known methods, for example, electroporation.

Appropriate expression vectors comprise regulatory sequences suitable for expression of DNA encoding a heterologous polypeptide or protein in a yeast host cell. Examples of regulatory sequences include promotors, operators, and enhancers, ribosomal binding sites, and sequences that control transcription and translation initiation and termination. The regulatory sequences may be operably linked to the DNA sequence to be expressed. For example, a promoter sequence is said to be operably linked to a coding sequence, if the promotor controls the transcription of the coding sequence.

The promoter may be any DNA sequence which shows transcriptional activity in yeast and may be derived from genes encoding proteins either homologous or heterologous to yeast. The promoter is preferably derived from a gene encoding a protein homologous to yeast. Examples of suitable promoters are the S. cerevisiae Mal, TPI, CUP, ADH or PGK promoters, or the P. pastoris glucose-6-phosphate isomerase promoter (PPGI), the 3-phosphoglycerate kinase promoter (PPGK) or glycerol aldehyde phosphate dehydrogenase promoter PGAP, the alcohol oxidase promoter (PAOX), formaldehyde dehydrogenase promoter (PFLD), isocitrate lyase promoter(PICL), translation elongation factor promoter (PTEF), and the promoters of P. pastoris enolase 1 (PENO1), triose phosphate isomerase (PTPI), alphaketoisocaproate decarboxylase (PTHI), ribosomal subunit proteins (PRPS2, PRPS7, PRPS31, PRPL1), heat shock protein family members (PSSA1, PHSP90, PKAR2), 6-Phosphogluconate dehydrogenase (PGND1), phosphoglycerate mutase (PGPM1), transketolase (PTKL1), phosphatidylinositol synthase (PPIS1), ferro-O2-oxidoreductase (PFET3), high affinity iron permease (PFTR1), repressible alkaline phosphatise (PPHO8), N-myristoyl transferase (PNMT1), pheromone response transcription factor (PMCM1), ubiquitin (PUBI4), single-stranded DNA endonuclease (PRAD2) and the promoter of the major ADP/ATP carrier of the mitochondrial inner membrane (PPET9).

The expression vector preferably comprises a yeast promoter, which contains a DNA sequence that functions as a promoter for gene transcription in yeast cells. Any suitable yeast promoter sequence may be employed. Suitable promoter sequences for yeast vectors include promoters for metallothionein, galactose, 3-phosphoglycerate kinase (Hitzeman et al., J. Biol. Chem. 255:2073, 1980) or other glycolytic enzymes (Hess et al., J. Adv. Enzyme Reg. 7:149, 1968, and Holland et al., Biochem. 17:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. The ADH2 promoter has been described by Russell et al. (J. Biol. Chem. 258:2674, 1982) and Beier et al. (Nature300:724, 1982).

A yeast expression vector preferably used according to the invention comprises a nucleotide sequence operably linked to a promoter, wherein the promoter is an inducible or a constitutive promoter. The promoter can be an endogenous promoter or heterologous to the host cell.

Promoter activity can be measured according to conventional methods. For example, an expression vector, in which a reporter gene is ligated downstream of a promoter such that the gene can be expressed, is constructed. Subsequently, a suitable host, e.g. yeast, is transformed with the expression vector. The obtained transformant is cultured under certain conditions, and the expression level of the reporter gene can be assayed at a level of mRNA or protein, so as to measure promoter activity under the above-described conditions.

The DNA sequence encoding the polypeptide of the invention may also be operably connected to a suitable terminator sequence, for example AOX1 (alcohol oxidase) terminator, CYC1 (cytochrome c) terminator, TEF (translation elongation factor) terminator.

Expression vectors may comprise one or more phenotypic selectable markers, e.g. a gene encoding a protein that confers antibiotic resistance or that supplies an autotrophic requirement, and an origin of replication recognized by the intended host cell to ensure amplification within the host. Yeast vectors commonly contain an origin of replication from a yeast plasmid, an autonomously replicating sequence (ARS), or alternatively, a sequence used for integration into the host genome, a promoter region, sequences for polyadenylation, sequences for transcription termination, and a selectable marker.

In another preferred embodiment, the yeast expression vector is able to stably integrate in the yeast genome, e. g. by homologous recombination.

In preferred embodiments, the nucleotide sequence of the xenogene is fused to a secretion signal, i.e. a peptide that targets protein to the secretory pathway where the signal peptide is cleaved and the protein released in the medium, for example alfa-factor, PHO or AGA-2.

Several approaches for the expression and secretion in yeast of proteins heterologous to yeast are preferred. Proteins are expressed, processed and secreted by transforming a yeast organism with an expression vector harbouring DNA encoding the desired protein and a signal peptide, preparing a culture of the transformed organism, growing the culture and recovering the protein from the culture medium. The signal peptide may be the desired protein's own signal peptide, a heterologous signal peptide or a hybrid of a native and a heterologous signal peptide. In the present context, the term "signal peptide" is understood to mean a presequence which is present as an N-terminal sequence on the precursor form of an extracellular protein expressed in yeast. The function of the signal peptide is to allow the heterologous protein to be secreted to enter the endoplasmatic reticulum. The signal peptide is normally cleaved off in the course of this process. The signal peptide may be heterologous or homologous to the yeast organism producing the protein. A preferred signal peptide is derived from the yeast alfa factor, acid phosphatase (*PHO1*) or from agglutinin (AGA-2).

A transformant host cell according to the invention obtained by transforming the yeast with the expression vector encoding the BIO1 and optionally further BIO genes surprisingly grows with or without the xenogene expression under normal growth conditions. In some cases, it is preferable to first cultivate the host cell line at conditions to grow efficiently to a large cell number without the burden of expressing a heterologous protein. When the cell line is prepared for the xenogene expression, cultivation techniques are chosen to produce the expression product.

When the transformant is grown with an inductive stimulus, a promoter may be activated to direct transcription of the gene under its control, and the gene product of the xenogene is expressed. Under growth conditions with an inductive stimulus, it usually grows slower than under normal conditions, but because it has already grown to a large cell number in the previous stage, the culture system as a whole produces a large amount of the heterologous protein. An inductive stimulus is preferably heat, or addition of cadmium, copper, an osmotic pressure increasing agent, hydrogen peroxide, ethanol, methanol, methylamine or the like.

According to the present invention it is surprisingly possible to effectively grow yeast cells without adding extra biotin, and at the same time provide for a host cell line for recombinant polypeptide or protein production.

For cultivating the host cell line in a bioreactor according to the invention usually a biotin-deficient medium is used, which does not contain essential amounts of biotin. Where biotin is not added to a medium, it is present only at very low concentrations in the cultivation medium for growing the host cell and/or expressing the xenogene, which cultivation medium preferably is devoid of exogenous or heterologous biotin. The biotin concentration is preferably less than 4*10⁻⁴ g/L, more preferably less than 1 *10⁻⁴ g/L, more preferably less than 2*10⁻⁵ g/L, or below the detection limit or even absent. The preferred biotin deficient medium thus contains less than 10⁻⁶ g/L. Though the host cell according to the invention is biotin prototrophic and able to grow without adding biotin to the cultivation medium, there may still be biotin levels detectable through the endogenous biotin produced, which may largely be consummated in the course of cell proliferation and growing to a certain extent.

The host cell according to the invention is preferably tested for its biotin prototrophy by the following growth test: Serial dilutions of yeast cultures (ranging from 10⁷ - 10³ cells/mL) are spotted on biotin-deficient minimal plates, and incubated at 30 °C for 2-3 days. Avidin is added to the media to ensure biotin-free conditions, and agarose is used for solid media instead of agar (which may contain traces of biotin). Growing colonies are then repeatedly cultivated on biotin-deficient plates to ensure a stable biotin-prototrophic phenotype.

It is preferred to cultivate the host cell line according to the invention under growth conditions to obtain a cell density of at least 1 g/L, more preferably at least 10 g/L cell dry weight, preferably at least 50 g/L cell dry weight. It is advantageous to provide for such yields of biomolecule production on a pilot or industrial scale.

The xenogene is preferably expressed employing conditions to produce at least 1 mg/L, preferably at least 10 mg/L, preferably at least 100 mg/L, most preferred at least 1 g/L.

Preferred fermentation techniques are batch, fed batch or continuous cultivation.

Preferably the yeast is cultivated in a mineral medium with a suitable carbon source, avoiding a vitamin cocktail, thereby further simplifying the isolation process significantly. An example of a preferred mineral medium is one containing an utilizable carbon source (e.g. glucose, glycerol or methanol), salts containing the macro elements (potassium, magnesium, calcium, ammonium, chloride, sulphate, phosphate) and trace elements (copper, iodide, manganese, molybdate, cobalt, zinc, and iron salts, and boric acid). A detailed fermentation media protocol can be found in example 7.

The transformed cells are cultivated under conditions suitable to effect expression of the desired recombinant compound, which can be purified from the cells or culture medium, depending on the nature of the expression system and the expressed protein, e.g. whether the protein is fused to a signal peptide and whether the protein is soluble or membrane-bound. As will be understood by the skilled artisan, cultivation conditions will vary according to factors that include the type of host cell and particular expression vector employed.

If the desired compound is secreted from the cells, it can be isolated and purified from the culture medium using state of the art techniques. Secretion of the recombinant expression products from the yeast cells is generally advantageous for reasons that include facilitating the purification process, since the products are recovered from the culture supernatant rather than from the complex mixture of proteins that results when yeast cells are disrupted to release intracellular protei ns.

Secreted proteins generally are initially expressed as precursors bearing an N-terminal signal or leader peptide. Signal peptides generally contain a positively charged N-terminus followed by a hydrophobic core, followed by a recognition site for an enzyme known as signal peptidase. This enzyme cleaves the signal peptide from the protein during translocation. The protein is transported from the endoplasmic reticulum to the Golgi apparatus, and then follows one of a number of routes in the secretory pathway, depending on the nature of the protein. The protein may be secreted into the culture medium or may be retained on the cell surface, for example. Certain receptors that comprise extracellular, transmembrane, and cytoplasmic domains are examples of proteins that may be retained on the cell membrane, with only the extracellular domain located outside the cell.

The cultured transformant cells may also be ruptured sonically or mechanically, enzymatically or chemically to obtain a cell extract containing the desired xenogene expression product, from which the desired product is isolated and purified.

As isolation and purification methods for obtaining a recombinant polypeptide or protein product, methods, such as methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be mentioned.

The isolated and purified compound can be identified by conventional methods such as Western blotting or assay of its activity. The structure of the purified compound can be defined by amino acid analysis, amino-terminal analysis, primary structure analysis, and the like. It is preferred that the compound is obtainable in large amounts and in a high purity level, thus meeting the necessary requirements for being used as an active ingredient in pharmaceutical compositions.

The preferred host cell line according to the invention maintains the integration of the BIO1 and xenogene, and the expression level remains high even, e.g. at least at a µg level, after about 20 generations of cultivation, preferably at least 30 generations, more preferably at least 40 generations, most preferred of at least 50 generations. The recombinant host cell is surprisingly stable, which is a great advantage when used for industrial scale protein production.

Further, the invention relates to the DNA construct described in the sequences illustrated in Sequ. ID No. 2 and Sequ. ID No. 3.

The present invention is described in further detail in the following examples, which are not in any way intended to limit the scope of the invention as claimed.

### Example 1: Biotin biosynthesis pathway in P. pastoris

In order to test if there are functional homologs of the biotin biosynthetic enzymes, the growth of *P. pastoris* X-33 (Invitrogen) was tested on SM-minimal medium as described below, containing DTB, DAPA or KAPA, respectively. SM-minimal medium contained per litre: 20 g glucose, 3.2 g (NH₄)₂HPO₄, 0.027 g CaCl₂ · 2H₂O, 0.8 g KCI, 0.5 g MgSO₄ · 7H₂O, 1.5 mL PTM1 trace salts stock solution, 1 mL vitamins stock solution (without biotin), 0.005% L-glutamic acid, L-methionine, L-lysine, L-leucine, and L-isoleucine, and 100 mM potassium phosphate buffer pH 6.0. The pH was set to 6.0 with 25% HCl. PTM1 trace salts stock solution contained per liter: 6.0 g CuSO₄ · 5H₂O, 0.08 g Nal, 3.36 g MnSO₄ · H₂O, 0.2 g Na₂MoO₄ · 2H₂O, 0.02 g H₃BO₃, 0.82 g CoCl₂ · 6H₂O, 20.0 g ZnCl₂, 65.0 g FeSO₄ · 7H₂O, and 5.0 mL H₂SO₄ (95-98%). Vitamins stock solution contained per liter: 0.2 g calcium pantothenate, 1 g inositol, 0.2 g niacin, 0.1 g p-aminobenzoic acid, 0.2 g pyridoxine hydrochloride, 0.1 g riboflavin, and 0.2 g thiamine hydrochloride. Avidin was added at a final concentration of 23 µg/mL to ensure biotin-free conditions when appropriate, and agarose (20 g/L) was used for solid media instead of agar (which may contain traces of biotin).

Two different concentrations of biotin and DTB were used, corresponding to the amounts commonly used for the cultivation of S. *cerevisiae* and *P. pastoris.* Interestingly, *P. pastoris* media recipes give up to 20-times higher concentrations of biotin (400 µg/L) as compared to *S. cerevisiae* (20-30 µg/L). Accordingly, DTB was employed at concentrations of 800 µg/L and 50 µg/L. Both *P. pastoris* X-33 and *S. cerevisiae* HA232 were able to grow on all the concentrations of biotin and DTB tested, however, only the *S. cerevisiae* strain was growing on KAPA (25 µg/L) and DAPA (5 µg/L).
These preliminary growth tests confirmed the presence of a *BIO2* homolog in P. *pastoris.*

### Example 2: Isolation of the biotin biosynthesis genes

The *BIO3, BIO4* and *BIO5* genes were amplified by PCR from genomic DNA of S. *cerevisiae* HA232, while the *BIO6* and the *BIO1* gene are derived from S. *cerevisiae*/*paradoxus* A364A. For subsequent cloning, all forward primers contain the Sbfl restriction site, and the backward primers the Sfil restriction site.

All isolated sequences were checked by DNA sequencing with the primers used for their amplification.

### Example 3: Construction of the BIOx expression vectors and expression in P. pastoris

For expression in *P. pastoris,* all biotin biosynthesis genes (subsequently referred to as *BIOx)* were cloned into the pPM2d expression vector (The sequence of the vector backbone including the TPI promoter is given in Sequ. ID No. 1), consisting of the pUC19 bacterial origin of replication, the *P. pastoris* glucose-6-phosphate isomerase promoter (P_{PGI}), the 3-phosphoglycerate kinase promoter (P_{PGK}), triose-isomerase promoter (P_{TPI}) or the glycerol aldehyde phosphate dehydrogenase promoter P_{GAP}, the S. *cerevisiae* CYC1 transcription terminator and the Zeocin antibiotic resistance cassette flanked by loxP sites (ZeoLox), as described in (Marx et al. 2008). Linearization of the expression vectors in the promoter regions allowed for integration into the *P. pastoris* genome. All the promoters can be exchanged for each other by digestion of the vectors with BstXI and Sbfl. The *BIO6* gene was additionally cloned into a vector containing the KanMX cassette from pFA6-KanMX4 (Wach et al. 1994) instead of the ZeoLox resistance marker. A complete list of the constructed expression vectors is given in Table 1.

**Table 1: Summary of the constructed expression vectors**

| **Short name** | **Resistance** | **Promoter** | **Gene** |
|---|---|---|---|
| pGAP_*BIO1* | ZeoLox | P_{GAP} | *BIO1* |
| pPGK_*BIO1* | ZeoLox | P_{PGK} | *BIO1* |
| pPGK_*BIO3* | ZeoLox | P_{PGK} | *BIO3* |
| pPGI_*BIO4* | ZeoLox | P_{PGI} | *BIO4* |
| pTPI_*BIO4* | ZeoLox | P_{TPI} | *BIO4* |
| pGAP_*BIO5* | ZeoLox | P_{GAP} | *BIO5* |
| pGAP_*BIO6* | ZeoLox | P_{GAP} | *BIO6* |
| pGAPKan_*BIO6* | KanMX | P_{GAP} | *BIO6* |
| pPGI_*BIO6* | ZeoLox | P_{PGI} | *BIO6* |

All plasmids were linearized prior to electrotransformation into *P. pastoris.*

Selection of positive transformants was performed on YPD (per liter: 10 g yeast extract, 20 g peptone, 20 g glucose, 20 g agar-agar) plates containing 25 µg/mL of Zeocin, and the integration of the respective *BIOx* gene was verified by colony PCR after digestion with lyticase (2.5 U; 1 h; 37 °C). Afterwards, selected clones were transformed with the transient Cre recombinase expression vector pKTAC-CRE, and plated on YPD containing 500 µg/mL of G418 (Marx et al. 2008). Subsequent cultivation without antibiotic was sufficient to cure the strains from the Cre plasmid. Clones that were not able to grow on Zeocin containing medium anymore, were checked for stable occurrence of the *BIOx* expression cassette(s) by colony PCR. For colony PCR, genomic DNA was gained by digestion of P. *pastoris* colonies with lyticase and directly applied for PCR with the appropriate primers.

The expression of the individual *BIO* genes from constitutive *P. pastoris* promoters was attempted. Using the procedure described above, pPGK_*BIO3* and pTPI_*BIO4* were integrated into the *P. pastoris* genome at the locus of the native promoters. The ZeoLox marker cassette was excised by transient expression of Cre recombinase, to allow subsequent selection steps with the Zeocin resistance. However, growth on the biotin precursor DAPA could not be achieved with these clones.
In a next step, for a proof of concept, a triple transformation with pGAP_*BIO1,* pGAP_*BIO5* and pGAP_*BIO6,* all linearized in the GAP promoter region, was performed. Transformants were plated both on 100 µg/mL Zeocin and on SM minimal agarose containing avidin to select for multiple gene integration. After repeated cultivation on selective media, colony PCR of 24 clones each was carried out, confirming integration of more than one gene in more than half of the analyzed clones. Interestingly, the majority of clones selected on Zeocin carries just one *BIO* gene, whereby different gene copy numbers may be possible, whereas selection on minimal medium resulted mainly in clones possessing copies of two different BIO genes (14 clones). Out of these, 13 clones feature the combination of *BIO1* and *BIO6* (see table 2).
Spotting of all these clones on biotin-deficient medium resulted in growth of five of the transformants (highlighted in yellow in table 2). While two of the strains have integrated all three missing BIO genes (*BIO1, BIO5* and *BIO6*), three others only had the *BIO1* and *BIO6* combination. After repeated streaking on selective SM-agarose plates, the yeasts were used to inoculate liquid SM medium. All 5 analyzed clones were able to grow without biotin different to the parental strain (carrying *BIO3* and *BIO4* only), irrespectively of the number of integrated BIO genes.

**Table 2: Integration of multiple BIO genes into the genome of P. pastoris verified by colony PCR**

| **clone** | ***BIO1*** | ***BIO6*** | ***BIO5*** | | **clone** | ***BIO1*** | ***BIO6*** | ***BIO5*** |
|---|---|---|---|---|---|---|---|---|
| Z1 | 0 | 1 | 1 | | 1 | 1 | 1 | 0 |
| Z2 | 0 | 1 | 0 | | 2 | 1 | 1 | 1 |
| Z3 | 0 | 0 | 0 | | 3 | 1 | 1 | 1 |
| Z4 | 1 | 0 | 0 | | **4** | **1** | **1** | **1** |
| Z5 | 0 | 1 | 0 | | 5 | 1 | 1 | 0 |
| Z6 | 0 | 0 | 0 | | 6 | 1 | 1 | 1 |
| Z7 | 1 | 0 | 0 | | 7 | 1 | 1 | 0 |
| Z8 | 0 | 1 | 1 | | **8** | **1** | **1** | **0** |
| Z9 | 1 | 0 | 0 | | **9** | **1** | **1** | **0** |
| **Z10** | **1** | **1** | **1** | | 10 | 1 | 1 | 0 |
| Z11 | 0 | 0 | 0 | | 11 | 1 | 1 | 0 |
| Z12 | 0 | 0 | 1 | | 12 | 1 | 0 | 1 |
| Z13 | 1 | 0 | 0 | | 13 | 0 | 1 | 1 |
| Z14 | 1 | 0 | 0 | | 14 | 1 | 1 | 0 |
| Z15 | 0 | 0 | 0 | | 15 | 0 | 0 | 0 |
| Z16 | 1 | 0 | 0 | | 16 | 1 | 1 | 0 |
| Z17 | 0 | 1 | 0 | | 17 | 1 | 1 | 0 |
| Z18 | 1 | 1 | 0 | | 18 | 1 | 0 | 0 |
| Z19 | 0 | 0 | 1 | | 19 | 1 | 1 | 0 |
| Z20 | 0 | 0 | 0 | | 20 | 1 | 1 | 1 |
| Z21 | 0 | 0 | 0 | | 21 | 0 | 1 | 0 |
| Z22 | 1 | 0 | 1 | | 22 | 0 | 1 | 0 |
| Z23 | 0 | 1 | 1 | | 23 | 1 | 0 | 0 |
| Z24 | 1 | 0 | 0 | | **24** | **1** | **1** | **0** |

Clones Z1-Z24 were selected on YPD containing 100 µg/mL Zeocin, whereas clones 1-24 were selected on SM-agarose containing avidin. 1 and 0 indicates the presence or absence of the respective gene in a P. pastoris clone. Clones highlighted in bold were able to grow without biotin both on plates as well as in liquid culture. All clones derive from a parental strain already containing pPGK_BIO3 and pTPI_BI04.

In order to prove that heterologous integration of four of the biotin biosynthesis genes is sufficient to render *P. pastoris* biotin-prototrophic, the parental strain carrying *BIO3* and *BIO4* was transformed with pGAPZ_*BIO1* and pGAPKan_*BIO6*. Selection was performed on YPD+Zeocin+G418. All of the transformants expressing the BIO genes from the constitutive promoter were able to grow when spotted on SM-agarose. Again, colony PCR was performed to verify the integration of the respective genes into the *P. pastoris* genome, and repeated streaking and liquid media cultivation of the clones assured their biotin prototrophic growth behavior.
Therefore we conclude that the KAPA transporter *BIO5* is not needed for biotin biosynthesis in *P. pastoris,* but probably only for the uptake of the precursor substance.

### Example 4: P. pastoris strains growing on biotin-deficient media

Synthetic minimal (SM) medium was inoculated with a single colony of a P. *pastoris* strain growing on SM+Avidin plates, and their parental strain as control. After five days of incubation (28 °C, 160 rpm), the yeast cell density was determined by measuring optical density at 600 nm. Aliquots of these cultures were transferred to fresh SM medium (at a standardized starting OD600 of 0.1), and incubation was carried out as described before. The results of the two subsequent cultivations are summarized in Table 3, where it can be easily seen that only the strains that have integrated the four heterologous biotin-biosynthetic genes *BIO1, BIO3, BIO4,* and *BIO6* can grow on medium lacking biotin, whereas the parental strains (carrying only *BIO3* and *BIO4*) are unable to grow.

**Table 3: Growth of biotin-prototrophic P. pastoris on biotin-deficient media**

| Optical density (OD600) of different P. pastoris clones after 5 days of cultivation on SM-medium at 28 °C with shaking. Clones 4, 8 and 24 are described in Table 2, clones K3-K9 additionally contain pGAPZ_BIO1 and pGAPKan_BIO6. The parental strain containing only pPGK_BIO3 and pTPI_BIO4 was used as control. | |
|---|---|
| **Clone** | **OD600** |
| **4** | 86 |
| **8** | 51.7 |
| **24** | 61.3 |
| **K3** | 60.1 |
| **K5** | 53.3 |
| **K6** | 59.8 |
| **K9** | 73.6 |
| **Control1** | 4.5 |
| **Control2** | 1.6 |
| **Control3** | 3.8 |

### Example 5: Construction of the BIOx multimer-expression vectors and expression in different strains of P. pastoris

Plasmids combining the expression cassettes for two *BIOx* genes on one vector were produced by digestion of one vector with *Mrel,* and the other vector with *Mrel* and *Agel* (producing complementary overhangs), and subsequent ligation of these constructs. Plasmids were linearized in either of the two present promoter sequences for targeted genome integration prior to electrotransformation into P. *pastoris.*
In a second approach, the Zeocin resistance cassette was exchanged for the ampicillin resistance marker from pUC19, to allow the generation of biotin-prototrophic yeast strains lacking a eukaryotic resistance marker.
Two different strains of P. pastoris, namely X-33 and DSMZ 70877 were cotransformed with pPM2d_pPGK_BIO3_pPGI_BIO4 (linearized in the PGK promoter) (Sequ. ID No. 3) and pPM2d_pPGI_BIO6_pPGK_BIO1 (linearized in the PGI promoter) (Sequ. ID No. 2).

While the zeocin containing clones were selected on both YPD containing 25 µg/mL Zeocin and biotin-deficient SM-minimal medium (containing 23 µg/mL avidin), the ampicillin containing clones only allowed for selection on the biotin-deficient plates. Biotin-prototrophic clones were subjected to confirmation by colony PCR and growth tests as described before.

### Example 6: Construction of a P. pastoris host cell line for the expression of recombinant trypsinogen

For the expression of a xenogene, transformation of the biotin-prototrophic P. pastoris strains is carried out with a plasmid containing the gene for porcine trypsinogen under control of the GAP promoter, with the S. cerevisiae alfa-mating factor sequence as secretion leader, and Zeocin as resistance marker (as described in Baumann et al. 2008. Biotech..Bioeng., 100(1):177-183).
After transformation clones are screened for the expression of porcine trypsinogen in shake flask cultivations on minimal medium containing 22.0 g citric acid, 3.15 g (NH4)₂HPO₄, 0.027 g CaCl₂·2H₂O, 0.8 g KCI, 0.5 g MgSO₄·7H₂O, 22 g glucose·1H₂O, 1.5 ml PTM1 trace salts stock solution. The pH is set to 5.5 with KOH.

The trypsinogen quantification is performed as described before (Hohenblum et al., 2004b) with a trypsin kinetic assay using p-tosyl-L-arginine methyl ester (TAME) after activation with bovine enterokinase.

### Example 7: Method of producing recombinant trypsinogen in a bioreactor

Fed batch cultivations are carried out in a 2.0 L working volume bioreactor (MBR; Wetzikon, Switzerland) with a computer based process control (ISE; Vienna, Austria). Fermentation temperature is controlled at 25°C, pH is controlled at 5.0 with addition of 25% ammonium hydroxide and the dissolved oxygen concentration is maintained above 20% saturation by controlling the stirrer speed between 600 and 1200 rpm, whereas the airflow is kept constant at 100 L h⁻¹. The media are as follows:
Batch medium containes per liter:
   2.0 g citric acid, 12.4 g (NH4)₂HPO₄, 0.022 g CaCl₂·2H₂O, 0.9 g KCI, 0.5 g MgSO₄·7H₂O, 40 g glycerol, 4.6 ml PTM1 trace salts stock solution. The pH is set to 5.0 with 25% HCl.
Glucose fed batch solution containes per liter:
   550 g glucose·1H₂O, 10 g KCI, 6.45 g MgSO₄·7H₂O, 0.35 g CaCl₂·2H₂O and 12 ml PTM1 trace salts stock solution. PTM1 composition is given in Example 1.

After approximately 27 h, the batch is finished and the glucose fed batch with a constant feed rate of 16 g h⁻¹ is started. The fermentations are terminated at approximately 100 h. Samples are taken frequently for the determination of biomass and the quantification of trypsinogen (as described in Example 6).

### Sequence IDs:

BIO1: DNA-Sequence: Sequ. ID No. 4; Protein Sequence: Sequ. ID No. 10
BIO2: DNA-Sequence: Sequ. ID No. 5; Protein Sequence: Sequ. ID No. 11
BIO3: DNA-Sequence: Sequ. ID No. 6; Protein Sequence: Sequ. ID No. 12
BIO4: DNA-Sequence: Sequ. ID No. 7; Protein Sequence: Sequ. ID No. 13
BIO5: DNA-Sequence: Sequ. ID No. 8; Protein Sequence: Sequ. ID No. 14
BIO6: DNA-Sequence: Sequ. ID No. 9; Protein Sequence: Sequ. ID No. 15

### Vectors used:

pPM2d expression vector: Sequ. ID No. 1
pPM2d_pPGI1_BIO6_pPGK1_BIO1 expression vector: Sequ. ID No. 2
pPM2d_pPGK1_BIO3 _pPGI1_BIO4 expression vector: Sequ. ID No. 3

## Claims

1. A host cell line of a biotin prototrophic yeast comprising a BIO1 gene and a xenogene.

2. A host cell line according to claim 1, wherein said yeast is a yeast of Saccharomycetales.

3. A host cell line according to any of claims 1-2, wherein said yeast is selected from the group consisting of Pichia, Saccharomyces, Kluyveromyces, Hansenula, Yarrowia and Komagataella.

4. A host cell line according to any of claims 1-3, further comprising at least one of a BIO gene selected from the group consisting of BIO2, BIO3, BIO4, BIO5, BIO6.

5. A host cell line according to any of claims 1-4, wherein said BIO1 or BIO gene is a heterologous gene.

6. A host cell line according to any of claims 1-5, whereby the host cell line is a Pichia pastoris comprising a BIO1, BIO2, BIO3, BIO4 and BIO6 gene, wherein at least one of the BIO genes is of Saccharomyces cerevisiae origin.

7. Biotin prototrophic Pichia pastoris.

8. Pichia pastoris according to claim 7, comprising a xenogene and/or a BIO1 gene.

9. A method of producing a host cell line of a biotin prototrophic yeast comprising a BIO1 gene and a xenogene, comprising the steps of:
a) providing a yeast host cell,
b) providing a BIO1 gene and a xenogene,
c) introducing said genes into said host cell, and
d) cultivating said host cell in a biotin-deficient medium to obtain a host cell line.

10. A method according to claim 9, wherein said genes are comprised in separate vectors.

11. A method according to claim 9, wherein said genes are comprised in a single vector.

12. A vector for use in a method according to any of claims 9 to 11, comprising a BIO1 gene and/or a gene coding for a xenogenic polypeptide.

13. A method of producing a product in a yeast host cell line, obtained by a method according to any of claims 9 to 11, **characterized in that** said xenogene is expressed, and the product is isolated.

14. A method according to claim 13, wherein said product is selected from the group consisting of serum proteins, such as immunoglobulins or serum albumin, enzymes, hormones, signaling molecules, matrix proteins, fragments or derivatives thereof.

15. A method according to claim 13, wherein said product is a metabolite of said host cell line.
